(19) Europäisches Patentamt
European Patent Office
Office européen des brevets

(11) EP 3 651 738 B1

# (12) EUROPEAN PATENT SPECIFICATION

(45) Date of publication and mention of the grant of the patent:
**03.04.2024 Bulletin 2024/14**

(21) Application number: **18749950.4**

(22) Date of filing: **13.07.2018**

(51) International Patent Classification (IPC):
***A61K 9/107*** (2006.01) ***A23L 2/395*** (2006.01)
***A23L 2/52*** (2006.01) ***A61K 9/16*** (2006.01)
***A61K 9/70*** (2006.01) ***A61K 31/05*** (2006.01)
***A61K 31/352*** (2006.01) ***A23L 29/00*** (2016.01)
***A61P 25/04*** (2006.01)

(52) Cooperative Patent Classification (CPC):
(C-Sets available)
**A61K 31/353; A23L 2/39; A23L 2/52; A23L 19/05; A23L 33/105; A23P 10/20; A61K 9/1075; A61K 9/1664; A61K 9/7007; A61K 31/05; A61K 31/352; A61K 36/185; A61P 25/04;**
A23V 2002/00 (Cont.)

(86) International application number:
**PCT/US2018/042150**

(87) International publication number:
**WO 2019/014631 (17.01.2019 Gazette 2019/03)**

## (54) CANNABINOID COMPOSITIONS AND METHODS OF PREPARATION THEREOF

CANNABINOIDHALTIGE ZUSAMMENSETZUNGEN UND DEREN HERSTELLUNG

COMPOSITIONS COMPRENANT DES CANNABINOÏDES ET LEURS PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.07.2017 US 201762532468 P**

(43) Date of publication of application:
**20.05.2020 Bulletin 2020/21**

(73) Proprietor: **5071, Inc.**
**Broomfield, CO 80020 (US)**

(72) Inventors:
- **WOELFEL, Keith**
  **Centennial, OH 80111 (US)**
- **SINGER, Justin**
  **Boulder, CO 80303 (US)**
- **GOLDSTEIN, Jeremy**
  **Boulder, CO 80304 (US)**
- **BROWN, Michael**
  **Aurora, CO 80011 (US)**

(74) Representative: **Cabinet Beau de Loménie**
**158, rue de l'Université**
**75340 Paris Cedex 07 (FR)**

(56) References cited:
**EP-A1- 1 437 136**
**WO-A1-2017/072762**
**CA-A1- 2 952 335**
**US-A1- 2015 045 282**
**US-A1- 2015 258 040**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/05, A61K 2300/00;**
**A61K 31/352, A61K 2300/00;**
A23V 2002/00, A23V 2200/31, A23V 2200/322, A23V 2250/194, A23V 2250/21, A23V 2250/5114, A23V 2250/5118, A23V 2250/642

**Description**

TECHNICAL FIELD

The present disclosure relates to ware-soluble cannabinoid compositions and use thereof, e.g., in foods and beverages.

BACKGROUND

Cannabis has a long history of being used for many purposes and in many forms. The psychoactive effects of cannabis may be well-known, however the medical benefits may be just as useful. Treating glaucoma, pain management, appetite stimulation and easing anxiety may be just a few of the potential benefits. The source of these effects may be in the cannabinoids, a class of compounds found in the cannabis plant. Tetrahydrocannabinol (THC) may be responsible for many of the psychoactive effects as well as the medicinal effects of cannabis. Cannabidiol is another major cannabinoid comprising up to 40% of cannabis extract and may also have certain health benefits.

Currently existing methods for extracting cannabinoids from the cannabis plant may be problematic. Because cannabinoid compounds are more soluble in non-polar liquids, extraction often includes alcohols or oil-based liquids. A common method includes alcohol extraction, which uses a solvent to extract the cannabinoids and then evaporate the alcohol, leaving a resin. Further extraction and evaporation may yield a product that is closer to a solid. Various other organic solvents may be used, however, liquids like hexane and methanol associated with health risks may not be appropriate in the preparation of food products.

Another method for the purposes of making edibles includes placing the cannabis leaves in butter, heavy cream, oil, etc., and then heating to extract the cannabinoids. However, such methods often result in high caloric foods and mixtures of cannabinoids that may provide adverse flavors. And cannabinoids formulated for use in foods are often solubilized in fat-carriers, such as oil-based products (e.g., oil, butter), or in alcohol, and subsequently be added to foods (e.g., brownies, cookies, muffins, etc.). However, fat-based carriers add significant calories to any products formed. These products may also lack versatility and utility as cannabinoid food enhancers.

For some food products, cannabinoids are entrapped in a crystalline matrix (e.g., sugar cubes, lollipops, etc.,) or gel matrix (e.g., gummies). While such sugarcubes temporarily entrap the cannabis, as the sugar dissolves the cannabinoid compounds can create "oil slicks" when added directly into water-based foods and beverages that negatively affect flavor and appearance. Similarly, fat-based carriers may be unsuitable for many foods and beverages that are water-based (e.g. coffee, tea, water, juice). With respect to tea, for example, placing cannabis leaves in hot water may not effectively extract vital cannabinoids. Since cannabinoids are not readily water soluble, adding cannabinoids to many foods and beverages can create an oil slick on the surface, providing an unappetizing appearance and taste. Further, products that typically contain fat-based cannabinoids (e.g., muffins, brownies, cookies) may be high in sugar, fat, and calories. Thus, those seeking a healthy lifestyle may not consider these foods to be functional for a day-to-day diet.

Another issue with incorporating cannabis into food products is adverse flavors that can result from some components of cannabis extract. Many products suffer from a strong, characteristic "green" or "skunk" taste that detracts from the quality and taste of the food or beverage.

WO 2017/072762 discloses *inter alia* a formulation comprising a plurality of particles of size of between about 10 and 200 nm, the nanoparticles comprising (a) a cannabinoid composition comprising a combination of cannabinoids, cannabinoid isoforms, derivatives, precursors or metabolites, (b) at least one surfactant, (c) at least one lipid component and (d) a water soluble biocompatible amphiphilic solvent.

SUMMARY

The present disclosure includes water-soluble cannabinoid compositions as defined below and methods of using such compositions.

The present disclosure includes water soluble cannabinoid compositions comprising a purified cannabinoid mixture, at least one carrier oil, and at least one water soluble agent chosen from a complex carbohydrate, a polyol, a polysaccharide, an oligosaccharide, or a combination thereof, wherein the composition is soluble in water at a temperature less than or equal to about 20°C, the composition being in the form of: agglomerated particles.

In some examples, the purified cannabinoid mixture is derived from cannabis plant matter and comprises less than 8.0%, less than 5.0%, or less than 1.0% by weight terpene compounds, such as from 0.1% to 5.0% by weight or from about 0.5% to about 3.0% by weight terpene compounds, optionally wherein the terpene compounds are chosen from β-myrcene, β-caryophyllene, limonene, linalool, α-bisabolol, α-pinene, β-pinene, caryophyllene oxide, terpinolene, phytol, or combinations thereof. The purified cannabinoid mixture may comprise at least 50% by weight, at least 70% by weight, or at least 85% by weight tetrahydrocannabinol (THC), cannabidiol (CBD), or a mixture thereof, and/or wherein

the purified cannabinoid mixture may comprise one or more of tetrahydrocannabivarin, cannabivarin, cannabigerol, cannabichromene, or cannabinol. Further, for example, the purified cannabinoid mixture may comprise CBD and THC in a weight ratio of CBD to THC ranging from about 60:1 to about 1:60, such as from about 50:1 to about 1:50, from about 40:1 to about 1:40, from about 30:1 to about 1:30, from about 25:1 to about 1:25, from about 20:1 to about 1:20, or from about 5:1 to about 1:5. According to some aspects, the composition comprises from about 0.05% to about 35.0% by weight, or from about 10.0% to about 30.0% by weight of the purified cannabinoid mixture.

In at least one example, the composition comprises at least one water soluble agent chosen from a starch such as a modified food starch, gum arabic, quillaja extract, a cyclodextrin, a sugar alcohol such as sorbitol or maltitol, maltodextrin, or a combination thereof. For example, the composition may comprise a modified food starch, sorbitol, or both. In some examples, the composition comprises at least two water soluble agents chosen from complex carbohydrates, polyols, polysaccharides, oligosaccharides, and combinations thereof. For example, the at least two water soluble agents may be chosen from starches such as modified food starches, sugar alcohols, quillaja extract, maltodextrin, or combinations thereof. In at least one example, the composition comprises a starch and a sugar alcohol. In some examples, the composition does not comprise one or more of maltodextrin, sorbitan, a sorbitan ester, or polysorbate. The composition may further comprise at least one antioxidant, flavoring agent, sweetener, coloring agent, food preservative, or combination thereof.

According to some aspects of the present disclosure, the weight ratio of the carrier oil(s) to the purified cannabinoid mixture may range from about 1:4 to about 2:1, or from about 1:2 to about 4:3. The carrier oil and the purified cannabinoid mixture may form a hydrophobic component of the composition, wherein the weight ratio of the at least one water soluble agent to the hydrophobic component ranges from about 1:5 to about 5:1, such as from about 1:4 to about 4:1, or from about 1:1 to about 2:1. The compositions herein may be vegan, sugar-free, nut-free, dairy-free, gluten-free, non-GMO, and/or clean label.

In some examples, the agglomerated particles have a bulk density ranging from about 0.2 g/cm$^3$ to about 0.7 g/cm$^3$, such as from about 0.4 g/cm$^3$ to about 0.7 g/cm$^3$. Additionally or alternatively, at least 40% by weight, such as at least 50% or at least 60% by weight, of the agglomerated particles have a particle size between about 150 $\mu$m and about 800 $\mu$m; and/or at least 60% by weight of the agglomerated particles have a particle size between 150 $\mu$m and 600 $\mu$m.

In some examples the composition has a Hausner ratio less than or equal to 1.25 and/or a compressibility index less than or equal to 20.

With respect to solubility, the composition (e.g., a 400 mg sample) may dissolve in water at a temperature less than or equal to 20°C within 30 seconds, within 25 seconds, within 20 seconds, within 15 seconds, or within 10 seconds. The composition may be more soluble than sucrose (table sugar), for example.

In some examples, the composition is sugar-free, nut-free, dairy-free, and/or gluten-free.

The compositions herein may be packaged as a sachet, a packet, a canister, or a bottle, among other containers. Further, for example, the compositions herein may be added to or incorporated into a food product or a beverage product. The food or beverage product may provide a cannabinoid dosage ranging from 0.5 mg/serving to 50.0 mg/serving, such as 1.0 mg/serving, 2.5 mg/serving, or 10 mg/serving.

Methods (not within the scope of the claims) of preparing the compositions herein include preparing emulsions, and in some cases, drying the emulsions. Preparing the emulsion may include combining the purified cannabinoid mixture with the carrier oil(s), water, and the water soluble agent(s), wherein the emulsion has a $d_{90}$ oil droplet size less than 10 $\mu$m, such as less than 6 $\mu$m, or less than 2 $\mu$m. The viscosity of the emulsion may be less than 500 cP, such as between 100 cP and 400 cP. In at least one example, preparing the emulsion includes dispersing the water soluble agent(s) (e.g., a complex carbohydrate) in water to hydrate the water soluble agent(s), and adding the purified cannabinoid mixture to the hydrated water soluble agent(s). In at least one example, preparing the emulsion comprises preparing a first emulsion having a $d_{90}$ oil droplet size greater than 2 $\mu$m; and reducing the droplet size of the first emulsion to form a second emulsion having a $d_{90}$ oil droplet size less than 2 $\mu$m, such as less than or equal to 800 nm, or less than or equal to 500 nm. further comprising drying the emulsion to a water moisture content of less than 10% by weight.

According to some aspects of the present disclosure, the method of preparing the composition (e.g., a film coating or a particulate composition) further comprises drying the emulsion to a water moisture content of less than 10% by weight, such as less than 8% by weight or less than 5% by weight. For example, drying the emulsion may include applying the emulsion to a substrate to form a film coating. Exemplary substrates include food products and beverage products such as, e.g., tea leaves, nuts, grains, seeds, dried fruit, dried vegetables, and combinations thereof. In some examples, drying the emulsion includes spray drying, tumble coating, or agglomeration.

Further provided herein are methods of using the compositions. For example, the composition may be used for reducing pain, reducing nausea, reducing inflammation, reducing stress, promoting sleep, preparing for and/or recovering from exercise, or boosting energy.

It is to be understood that both the foregoing general description and the following detailed description are exemplary and explanatory only and are not restrictive of the detailed embodiments, as claimed.

DETAILED DESCRIPTION

Particular aspects of the present disclosure are described in greater detail below. The terms and definitions provided herein control, if in conflict with terms and/or definitions incorporated by reference.

As used herein, the terms "comprises," "comprising," or any other variation thereof are intended to cover a non-exclusive inclusion, such that a process, method, composition, article, or apparatus that comprises a list of elements does not include only those elements, but may include other elements not expressly listed or inherent to such process, method, composition, article, or apparatus. The term "exemplary" is used in the sense of "example" rather than "ideal."

As used herein, the singular forms "a," "an," and "the" include plural reference unless the context dictates otherwise. The terms "approximately" and "about" refer to being nearly the same as a referenced number or value. As used herein, the terms "approximately" and "about" should be understood to encompass $\pm$ 5% of a specified amount or value.

Described herein are compositions comprising one or more cannabinoid compounds suitable for incorporating into a variety of foods and beverages. The compositions are in the form of agglomerated particles and may be at least partially water soluble. For example, the compositions may be soluble in room temperature water (e.g., water at a temperature of about 20°C) and in cold water (e.g., water at a temperature less than about 20°C, such as 5°C). The methods herein are suitable for large-scale production of water soluble cannabinoid compositions.

The compositions herein comprise one or more cannabinoid compounds, e.g., in a purified cannabinoid mixture, at least one carrier oil, at least one water soluble agent chosen from a complex carbohydrate (which may at least partially absorb the cannabinoid compound(s) and/or carrier oil(s)), a polyol, a polysaccharide, an oligosaccharide, or a combination thereof. The composition is in the form of agglomerated particles. The compositions herein may be water soluble, clean in taste (e.g., devoid of a bitter taste) and translucent in appearance, allowing foods and beverages to retain their flavors when the composition is added.

The term "cannabinoid" generally refers to a compound that acts on the cannabinoid receptor. Examples of cannabinoid compounds that may be provided in the compositions herein include, but are not limited to, tetrahydrocannabinol (THC), cannabidiol, cannabigerol, cannabichromene, cannabicyclol, cannabivarin, cannabielsoin, cannabicitran, cannabigerolic acid, cannabigerolic acid monomethylether, cannabigerol monomethylether, cannabigerovarinic acid, cannabigerovarin, cannabichromenic acid, cannabichromevarinic acid, cannabichromevarin, cannabidolic acid, cannabidiol monomethylether, cannabidiol-C4, cannabidivarinic acid, cannabidiorcol, delta-9-tetrahydrocannabinolic acid A, delta-9-tetrahydrocannabinolic acid B, delta-9-tetrahydrocannabinolic acid-C4, delta-9-tetrahydrocannabivarinic acid, delta-9-tetrahydrocannabivarin, delta-9-tetrahydrocannabiorcolic acid, delta-9-tetrahydrocannabiorcol, delta-7-cis-isotetrahydrocannabivarin, delta-8-tetrahydrocannabiniolic acid, delta-8-tetrahydrocannabinol, cannabicyclolic acid, cannabicylovarin, cannabielsoic acid A, cannabielsoic acid B, cannabinolic acid, cannabinol methylether, cannabinol-C4, cannabinol-C2, cannabiorcol,10-ethoxy-9-hydroxy-delta-6a-tetrahydrocannabinol, 8,9-dihydroxy-delta-6a-tetrahydrocannabinol, cannabitriolvarin, ethoxycannabitriolvarin, dehydrocannabifuran, cannabifuran, cannabichromanon, cannabicitran, 10-oxo-delta-6a-tetrahydrocannabinol, delta-9-cistetrahydrocannabinol, 3, 4, 5, 6-tetrahydro-7-hydroxy-alpha-alpha-2-trimethyl-9-npropyl-2, 6-methano-2H-1-benzoxocin-5-methanol-cannabiripsol, trihydroxy-delta-9-tetrahydrocannabinol, cannabinol, and derivatives thereof.

Tetrahydrocannabinol (THC) has the following chemical structure:

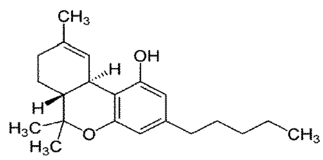

THC (and/or derivatives of THC) is generally understood to target CB1 receptors in the brain with certain psychoactive effects. THC may provide various health benefits, such as decreasing pain and/or nausea, promoting sleep, and/or reducing stress disorder. Studies suggest that lower doses of THC may provide positive health effects without negative effects such as anxiety and paranoia that can be present at higher, e.g., chronic, use levels.

Cannabidiol has the following chemical structure:

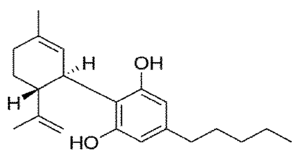

Cannabidiol (CBD) (and/or derivatives of CBD) is generally understood to act on CB2 receptors throughout the body and is associated with non-psychoactive effects. When used in combination with THC, CBD may help to counteract the psychoactivity of THC. CBD may provide various health benefits such as reduction in anxiety, sleep loss, blood sugar, pain, and/or inflammation; and/or may assist in treatment for various diseases or medical conditions such as multiple sclerosis, epilepsy, and Alzheimer's Disease.

Tetrahydrocannabivarin is a cannabinoid having approximately twice the psychoactive effect of THC, but with half the shelf life. It is associated with various health benefits, such as, e.g., boosting energy, and suppressing appetite, anxiety and/or stress. Cannabivarin is a non-psychoactive cannabinoid that may help reduce seizures. Cannabigerol is also non-psychoactive cannabinoid, which may provide certain health benefits such as increased brain and bone growth, anti-bacterial effects, and/or reduced insomnia. Cannabichromene is a non-psychoactive cannabinoid that may also act as an anti-inflammatory and anti-viral agent, and also may be more powerful than cannabidiol in reducing anxiety and stress. Cannabinol is a mild to non-psychoactive cannabinoid, a product of THC degradation that may act as a strong sedative. Cannabinol also may be useful in the treatment of insomnia, glaucoma and pain.

In at least one example, the composition may comprise one or more of THC (e.g., delta 9 tetrahydrocannabinol), CBD, tetrahydrocannabivarin, cannabivarin, cannabigerol, cannabichromene, cannabinol, a derivative of any of the foregoing, or a combination thereof. For example, the composition may comprise a purified cannabinoid mixture comprising at least 50%, at least 60%, at least 70%, at least 80%, at least 85%, at least 90%, or at least 95% by weight of THC, CBD, or a mixture thereof, and/or the purified cannabinoid mixture comprises one or more of tetrahydrocannabivarin, cannabivarin, cannabigerol, cannabichromene, or cannabinol. Further, for example, the composition may comprise a purified cannabinoid mixture comprising at least 50%, at least 60%, at least 70%, at least 80%, at least 85%, at least 90%, or at least 95% by weight of CBD and optionally one or more of THC, tetrahydrocannabivarin, cannabivarin, cannabigerol, cannabichromene, or cannabinol. In some examples, the composition may comprise a purified cannabinoid mixture comprising at least 50%, at least 60%, at least 70%, at least 80%, at least 85%, at least 90%, or at least 95% by weight of tetrahydrocannabivarin, cannabivarin, cannabigerol, cannabichromene, or cannabinol. The term CBD isolate generally refers to a cannabinoid oil comprising greater than 98% by weight CBD. The term THC distillate generally refers to a cannabinoid oil comprising from about 70% by weight to about 90% by weight or more THC.

The cannabinoid compound(s) may be derived (e.g., isolated, extracted, distilled, processed, etc.) from a natural cannabis source, such as cannabis plant matter or an extract thereof. Raw or crude cannabis extracts generally comprise hundreds of chemical compounds. According to some aspects of the present disclosure, the cannabinoid extract may be purified, e.g., to remove or reduce the concentration of certain compounds present in the natural cannabis plant matter and/or to enrich the concentration of certain compounds relative to others. For example, a raw or crude cannabis extract may be processed in one or more steps to remove extraneous plant matter and/or to remove or reduce the concentration of certain compounds associated with an unpleasant or unsavory flavor, smell, and/or bitterness.

For example, raw cannabis extracts can comprise 100-200 different types of terpene compounds, at least some of which can provide a pungent smell or bitter taste characteristic of raw or unrefined cannabis materials and products. This adverse smell/taste is sometimes described as "green" or "skunky," and can detract from a user's satisfaction with the product. On the other hand, certain terpene compounds may have health benefits or may contribute to a pleasant flavor or smell when present in the composition in controlled amounts. In some embodiments, one or more terpene compounds may be retained in the purified cannabinoid extract or mixture. Removal and/or retention of terpenes in the purification process may be selected to balance their purported or potential health effects while controlling and/or reducing an unsavory flavor or smell.

Examples of terpene compounds that may be retained in the purified cannabinoid mixtures of the composition herein include, but are not limited to, β-myrcene, β-caryophyllene, limonene, linalool, α-bisabolol, α-pinene, β-pinene, caryophyllene oxide, terpinolene, and phytol. For example, β-myrcene may have antioxidant and/or anticarcinogenic properties, and is believed to help regulate sleep and manage pain, inflammation, depression, and muscle tension. Further, β-caryophyllene has been associated with gastroprotective and anti-inflammatory effects, and may assist in the treatment of ulcers and arthritis. Limonene has a characteristic citrus aroma. Linalool may assist in stress reduction, sleep, and anti-inflammation, while bisabolol may have antioxidant, anti-inflammatory, antimicrobial, and/or analgesic properties, α- and β-pinene have a characteristic pine/evergreen aroma, and may assist with alertness, anti-inflammatory properties, and in memory retention. Caryophyllene oxide may have anti-fungal and/or anti-bacterial properties, and may help to combat insomnia. Phytol is a compound common to aged green teas and may serve as a sleep aid.

According to some aspects of the present disclosure, a cannabis extract may be purified in one or more processes to remove select compounds or a portion thereof from the extract. For example, the cannabis extract may undergo one or more extraction, solubilization, winterization, filtration, and/or distillation processes, e.g., to separate or remove portions of the extract based on chemical and/or physical properties.

For example, a cannabis source (e.g., plant matter) may be used to prepare a feedstock for the cannabinoid mixture. In some embodiments, the preparation of the feedstock may include drying and then reducing the particle size of the cannabis source (e.g., crushing or grinding plant matter) to improve extraction efficiencies. The feedstock then

may be extracted by a suitable solvent to yield a crude liquid (e.g., oil) extract comprising cannabinoid and other compounds. For example, supercritical $CO_2$ may be used for the extraction process, wherein the remaining spent feedstock may be discarded as waste. The crude oil then may be further purified. For example, the crude extract may be winterized to remove extraneous plant material, waxes, lipids, and/or fat. Purification may additionally or alternatively include filtration (e.g., to remove or reduce insoluble plant matter. In some aspects of the present disclosure, the extract may be heated, e.g., to activate THC through decarboxylation of tetrahydrocannabinolic acid (THCA). Further, purification of the cannabis extract may include one or more iterations of distillation (e.g., 2, 3, or more distillation steps) to remove select components of the extract, such as unwanted terpenes that may impart an undesired smell or flavor. In at least one example, the extract may be triple distilled, e.g., to sequentially remove portions of the extract by volatility to yield a purified distillate. It is contemplated that purification, and the removal and/or reduction of impurities or unwanted components, such as terpenes, may result in a bland taste. The above described purification process(es) may remove a significant amount of the undesirable bitter flavors and/or smell associated with cannabis products while retaining at least some of the terpenes that may provide various health benefits.

The purified cannabinoid mixture may comprise from 0 to 99.9% by weight of one of more cannabinoid compounds. The term mixture should not be understood as requiring certain combinations of cannabinoids and/or other compounds. For example, a substantially pure THC or CBD oil (e.g., greater than 99.0%, greater than 99.5%, or greater than 99.9% by weight THC or CBD) is generally encompassed by the term purified cannabinoid mixture. According to some aspects of the present disclosure, the purified cannabinoid mixture comprises from 0 to 99.9% of a cannabinoid compound chosen from THC, CBD, tetrahydrocannabivarin, cannabivarin, cannabigerol, cannabichromene, or cannabinol.

According to some aspects of the present disclosure, the purified cannabinoid mixture may comprise from 0 to 99.9% by weight THC and/or from 0 to 99.9% by weight CBD. In some examples, the purified cannabinoid mixture may comprise at least 50%, at least 65%, at least 75%, at least 80%, or at least 90% by weight THC, CBD, or a mixture thereof, with respect to the total weight of the purified cannabinoid mixture. In the case of mixtures of CBD and THC, the weight ratio of CBD to THC may range from about 30:1 to about 1:30, such as from about 25:1 to about 1:25, from about 20:1 to about 1:20, from about 15:1 to about 1:15, from about 10:1 to about 1:10, from about 5:1 to about 1:5, from about 1:2 to about 2:1, or about 1:1.

For example, the purification processes herein may provide a purified cannabinoid mixture comprising at least 50% by weight THC, such as at least 55%, at least 60%, at least 65%, at least 70%, at least 75%, at least 80%, at least 85%, at least 90%, at least 95%, or at least 97% by weight THC, e.g., from 50% to about 99% by weight THC, from 50% to about 90% by weight THC, from about 60% to about 99% by weight THC, from about 75% to about 95% by weight THC, from about 80% to about 99% by weight THC, from about 85% to about 95% by weight THC, or from about 90% to about 95% by weight THC. The purified cannabinoid mixture also may comprise from about 0.01% to about 10.0% by weight of other cannabinoid compounds, such as, e.g., CBD, tetrahydrocannabivarin, cannabivarin, cannabigerol, cannabichromene, and/or cannabinol. For example, the purified cannabinoid mixture may comprise from 0.01% by weight to about 5.0% by weight, from about 0.5% by weight to about 2.0% by weight, from about 2.0% by weight to about 4.0% by weight, or from about 2.5% by weight to about 3.5% by weight of cannabinoid compounds other than THC. In at least one example, the purified cannabinoid mixture comprises about 85% by weight THC and 0.5% or less by weight CBD.

Further, for example, the purification processes herein may provide a purified cannabinoid mixture comprising at least 50% by weight CBD, such as at least 55%, at least 60%, at least 65%, at least 70%, at least 75%, at least 80%, at least 85%, at least 90%, at least 95%, or at least 97% by weight CBD, e.g., from 50% to about 99% by weight CBD, from about 60% to about 99% by weight CBD, from about 75% to about 95% by weight CBD, from about 80% to about 99% by weight CBD, from about 85% to about 95% by weight CBD, or from about 90% to about 95% by weight CBD. The purified cannabinoid mixture also may comprise from about 0.01% to about 10.0% by weight of other cannabinoid compounds, such as, e.g., THC, tetrahydrocannabivarin, cannabivarin, cannabigerol, cannabichromene, and/or cannabinol. For example, the purified cannabinoid mixture may comprise from 0.01% by weight to about 5.0% by weight, from about 0.5% by weight to about 2.0% by weight, from about 2.0% by weight to about 4.0% by weight, or from about 2.5% by weight to about 3.5% by weight of cannabinoid compounds other than CBD.

In yet other examples, the purified cannabinoid mixture comprises from 50% to about 99% by weight of tetrahydrocannabivarin, cannabivarin, cannabigerol, cannabichromene, or cannabinol, such as from about 60% to about 99% by weight, from about 75% to about 95% by weight, from about 80% to about 99% by weight, from about 85% to about 95% by weight, or from about 90% to about 95% by weight of tetrahydrocannabivarin, cannabivarin, cannabigerol, cannabichromene, or cannabinol.

According to some aspects of the present disclosure, the purified cannabinoid mixture may comprise less than 8.0% by weight terpene compounds (including e.g., terpenoid compounds), such as less than 5.0% by weight, less than 3.0% by weight, less than 1.0% by weight, or less than 0.5% by weight. As mentioned above, it may be desirable to retain at least a portion of the terpene compounds of the crude cannabis extract. Thus, in some examples, the purified

cannabinoid extract may comprise from about 1.0% to about 8.0% by weight terpene compounds, such as from about 0.1% to about 5.0% by weight, or from about 0.5% to about 3.0% by weight terpene compounds. Exemplary terpene compounds that may be present in the purified cannabinoid mixture include, but are not limited to, β-myrcene, β-caryophyllene, limonene, linalool, α-bisabolol, α-pinene, β-pinene, caryophyllene oxide, terpinolene, and phytol, and combinations thereof.

The purified cannabinoid mixture is combined with one or more carrier oils, such as medium chain triglyceride (MCT) oil, long chain triglyceride (LCT) oil, vegetable oil, canola oil, olive oil, sunflower oil, coconut oil (including fractionated coconut oil), hemp oil, palm oils, and/or other oils suitable for consumption. In some cases, the addition of one or more carrier oils may help to improve solubility of the cannabinoid compounds and/or facilitate homogeneous dispersion of the cannabinoid compound(s) into the hydrophilic component or water soluble matrix formed by water and at least one water soluble agent (discussed below). Further, for example, the carrier oil(s) may be useful to increase the stability of the oil-in-water emulsion, e.g., including for higher levels of cannabinoids. Coconut oil is noted for a high saturated, MCT content. Hemp oil comprises about 80% essential fatty acids and is obtained from hemp seeds, which come from a variety of the Cannabis sativa plant that does not contain a high amount of THC. If desired, the carrier oil may be purified beforehand, or the combined cannabinoid/carrier oil mixture may be purified according to one or more processes as described above. Together, the carrier oil and the purified cannabinoid mixture may form a hydrophobic component of the composition. In some aspects of the present disclosure, the purified cannabinoid mixture may be used as a hydrophobic component of the composition with the addition of a carrier oil. In some examples, the weight ratio of carrier oil to purified cannabinoid mixture (carrier oil:mixture) may range from about 1:100 to about 10:1, such as from about 1:50 to about 5:1, from about 1:10 to about 2:1, from about 3:4 to about 4:3, or from about 1:2 to about 1:1, e.g., a ratio of about 10:1, 5:1, 3:1, 2:1, 4:3, 1:1, 3:4, 1:2, 1:3, 1:5, 1:10, 1:15, 1:20, 1:25, 1:50, 1:75, or 1:100. In some examples, the weight ratio of carrier oil to purified cannabinoid mixture may range from about 1:4 to about 2:1, from about 1:2 to about 4:3, or from about 1:1 to about 2:1.

In some examples, the composition does not include a carrier oil such as MCT oil, vegetable oil, canola oil, olive oil, sunflower oil, coconut oil, hemp oil, or palm oil. For example, the hydrophobic component of the composition may consistent essentially of, or may consist of, the purified cannabinoid mixture without any other oil(s).

The composition further comprises a hydrophilic component, e.g., comprising one or more water soluble agents chosen from a complex carbohydrate such as a starch, gum arabic, and quillaja extract; an oligosaccharide (e.g., acyclodextrin), a polysaccharide (e.g., maltodextrin); a polyol including, e.g., sugar alcohols such as sorbitol and maltitol, or a combination thereof. Additional water soluble agents that may be used herein include proteins (e.g., gelatin, whey, casein), phospholipids (e.g., soy lecithin, egg lecithin, etc.), glycerol monostearate, surfactants (such as, e.g., sorbitan, sorbitan esters, and polysorbates (e.g., sorbitan monolaurate, polyoxyethylene (20) sorbitan monolaurate, sorbitan monopalmitate, polyoxyethylene (20) sorbitan monopalmitate, sorbitan monostearate, polyoxyethylene (20) sorbitan monostearate, sorbitan tristearate, sorbitan monooleate, polyoxyethylene (20) sorbitan monooleate, etc.), and other emulsifiers and water soluble agents suitable for human consumption. The water soluble agent(s) may have a chemical structure that includes a hydrophilic region to promote solubility. Without intending to be bound by theory, it is believed that the water soluble agent(s) may promote solubility of the cannabinoid compounds, e.g., by at least partially absorbing the cannabinoid compounds or otherwise associating the cannabinoid compounds with hydrophilic portions of the water soluble agent.

In some examples, the water soluble agent may comprise one or more complex carbohydrates, including e.g., natural carbohydrates such as starches, gum arabic, and quillaja extract. The starch may be a food starch (e.g., waxy maize, corn, potato, wheat, tapioca, or cassava, etc.), and may be relatively high in amylopectin and/or chemically modified to increase an oil absorption capacity of the starch. Examples of starches suitable for the compositions herein include different types of modified food starches, including, but not limited to, octenyl succinic anhydride (OSA) starch. In some examples, the composition may comprise at least one complex carbohydrate in combination with one or more other water soluble agents, such as, e.g., oligosaccharides, polysaccharides, surfactants, and/or polyols. Further, for example, the composition may comprise two or more different complex carbohydrates, optionally in combination with one or more oligosaccharides, polysaccharides, surfactants, and/or polyols. Commercial examples of water soluble agents suitable for the compositions and methods herein include, but are not limited to, CAPSUL® starch, PURITY GUM® starch, N-ZORBIT® starch, PENBIND® starch, N-Lite® LP starch, and Q-Naturale® quillaja extract produced by Ingredion; and Span® 20, Span® 40, Span® 60, Span® 80, Tween® 20, Tween® 40, Tween® 60, and Tween® 80, produced by Croda International PLC.

The composition comprises at least one water soluble agent chosen from a complex carbohydrate, a polyol, a polysaccharide, an oligosaccharide, or a combination thereof. For example, the water soluble agent(s) may comprise a starch, quillaja extract, maltodextrin, a sugar alcohol, or a combination thereof. In at least one example, the water soluble agent(s) comprise a modified food starch, sorbitol, or both. According to some aspects of the present disclosure, the composition comprises at least two water soluble agents. For example, the composition may comprise two or more different water soluble agents chosen from complex carbohydrates, polyols, polysaccharides, oligosaccharides, and

combinations thereof. Further, for example, the two or more different water soluble agents may be chosen from modified food starches, sugar alcohols, quillaja extract, maltodextrin, or combinations thereof. In some examples, the two different water soluble agents comprise a starch and a sugar alcohol.

Certain water soluble agent(s) may provide sweetness to the composition. For example, sorbitol is a sugar alcohol that is generally understood to be metabolized at a slower rate than sugar, and thus may be described as a sugar substitute. Further, for example, maltodextrin is a long-chain polysaccharide that may be described as moderately sweet. In general, a longer chain length corresponds to a composition with less sweetness. For example, the water soluble agent(s) may comprise a polysaccharide or oligosaccharide that does not provide any sweetness, e.g., a polysaccharide or oligosaccharide that is flavorless.

In some examples herein, the weight ratio of water soluble agent(s) to hydrophobic component (i.e., purified oil distillate and carrier oil(s)) may range from about 10:1 to about 1:100, such as from about 5:1 to about 1:50, from about 4:1 to about 1:20, from about 3:1 to about 1:15, from about 2:1 to about 1:10, or from about 4:1 to about 1:4, e.g., a ratio of about 10:1, 5:1, 4:1, 3:1, 5:2, 2:1, 4:3, 1:1, 3:4, 1:2, 2:5, 1:3, 1:4, 1:5, 1:10, 1:25, 1:50, 1:75, or 1:100. In some examples, the weight ratio of water soluble agent(s) to the hydrophobic component ranges from about 1:5 to about 2:1, e.g., a weight ratio of up to about 1:1, up to about 1:2, up to about 1:3, up to about 1:4, or up to about 1:5.

In some aspects of the present disclosure, the composition may comprise one or more natural or artificial sweeteners such as sugars, sugar alcohols, sugar substitutes, or a combination thereof (some of which also may serve as a water soluble agent). Exemplary sweeteners include, but are not limited to, monosaccharides (e.g., fructose), disaccharides (e.g., sucrose), polysaccharides, stevia, turbinado, agave syrup, monk fruit, sorbitol, sucralose, aspartame, saccharin, xylitol, and erythritol. In some examples, the compositions may be sugar free. The term "sugar free" refers to a composition having less than 0.5 g of sugar per serving. Some sugar free compositions according to the present disclosure may comprise, for example, one or more sweeteners that are metabolized more slowly or differently than sucrose (table sugar). Some compositions herein do not comprise any sweeteners (natural or artificial), and therefore may also be described as sugar free. In at least one example, the composition comprises one or more natural sweeteners and does not comprise any artificial sweeteners.

The compositions herein may comprise one or more other ingredients or agents, such as, e.g., an antioxidant and/or other stabilizing or food preservation agent, a flavoring agent, a coloring agent, or a combination thereof, any of which may be natural or synthetic. Stabilizing and food preservation agents include substances that promote or maintain the physical and/or chemical stability of a mixture of various components (e.g., hydrophobic component comprising the cannabinoid mixture and the hydrophilic component comprising the water soluble agent(s) and/or food or beverage product). Non-limiting examples of food preservation agents include benzoates (e.g., sodium benzoate, benzoic acid, etc.), sorbates (e.g., sodium sorbate, potassium sorbate, sorbic acid, etc.), and citric acid. Stabilizing agents further include, for example, antioxidants, which may inhibit degradation of cannabinoids or various components (e.g., flavoring agents) of the mixture over time, thus extending shelf-life. Antioxidants suitable for the compositions herein include, but are not limited to carnosic acid and substances that contain carnosic acid, such as rosemary extract, as well as tertiary butylhydroquinone (TBHQ), butylated hydroxytoluene (BHT), butylated hydroxyanisole (BHA), propyl gallate, flavonoids, carotenoid terpenoids (e.g., lutein, beta-carotene), phenolic acids (e.g., cinnamic acids), and certain vitamins, such as vitamin C (ascorbic acid), vitamin E (e.g., tocopherols) and vitamin A. Antioxidants may also provide salutary benefits to the composition.

Flavoring agents include compounds that add flavor, such as, e.g., amyl acetate, benzaldehyde, ethyl butyrate, methyl anthranilate, methyl salicylate, fumaric acid, diacetyl, cinnamaldehyde, ethyl 25 propionate, limonene, ethyl decadienoate, allyl hexanoate, ethyl maltol, ethylvanillin, and methyl salicylate, among other examples. Flavoring agents refers to substances other than compounds that are retained from the cannabis extract (e.g., limonene may be added as a flavoring agent in addition to limonene that may be retained in the purified cannabinoid mixture from the cannabis extract). Other suitable flavors include bitter maskers/bitter blockers and sweetness enhancers. Coloring agents include substances that add or change the color to a composition, such as dyes or pigments. Such coloring agents may come in many forms, including liquids, powders, gels, dyes, lakes, and pastes. In some examples herein one or more coloring agents may be added to adjust the appearance of the composition, e.g., to be more appealing to the consumer.

The compositions herein may comprise from 0% to about 95.0% by weight of a complex carbohydrate, such as a modified food starch, from 0% to 99.0% by weight of a polysaccharide, from 0% to about 50.0% of a carrier oil, from about 0.5% to about 90.0% by weight of a purified cannabinoid mixture (e.g., the purified cannabinoid mixture comprising from 50.0% to 95.0% of THC, CBD, tetrahydrocannabivarin, cannabivarin, cannabigerol, cannabichromene, or cannabinol), and from about 0.5% to about 10.0% by weight water. For example, the compositions herein may comprise from 0.1% to 50.0% by weight of a complex carbohydrate, from 0.1% to 50.0% of a polysaccharide, from 0.1% to 50.0% of a carrier oil, from 0.1% to 50.0% of a purified cannabinoid mixture (e.g., the purified cannabinoid mixture comprising from 75.0% to 90.0% of THC, CBD, tetrahydrocannabivarin, cannabivarin, cannabigerol, cannabichromene, or cannabinol), and from 2.5% to 5.5% by weight water. Such compositions may optionally comprise an antioxidant.

According to some aspects of the present disclosure, the composition may comprise from about 0.05% to about

60% by weight of a purified cannabinoid mixture as described herein, such as from about 0.1 % to about 55.0% by weight, from about 0.5% to about 50.0% by weight, from about 1.0% to about 40.0% by weight, from about 2.5% to about 35.0% by weight, from about 5.0% to about 30.0% by weight, from about 7.5% to about 25.0% by weight, from about 10.0% to about 20.0% by weight, from about 5.0% to about 15.0% by weight, or from about 25.0% to about 50.0% by weight with respect to the total weight of the composition. For example, the compositions herein may comprise about 0.1%, about 0.5%, about 1.0%, about 5.0%, about 10.0%, about 12.5%, about 15.0%, about 17.5%, about 20.0%, about 22.5%, about 25.0%, about 27.5%, about 30.0%, about 32.5%, about 35.0%, about 37.5%, about 40.0%, about 42.5%, about 45.0%, about 47.5%, about 50.0%, or greater than 50.0% by weight of the purified cannabinoid mixture, with respect to the total weight of the composition.

Additionally or alternatively, the composition may comprise from about 0.1% to about 80.0% by weight water soluble agent(s), such as from about 1.0% to about 75.0% by weight, from about 5.0% to about 70.0% by weight, from about 10.0% by weight to about 60.0% by weight, from about 20.0% by weight to about 50.0% by weight, or from about 40.0% to about 50.0% by weight, with respect to the total weight of the composition. The water soluble agents may comprise one, two, or three or more different types of water soluble agents. Such water soluble agents may comprise, for example, one or more of a carbohydrate (including e.g., one or more complex carbohydrates), a polysaccharide, a polyol, or a combination thereof. In some examples, the composition is or comprises agglomerated particles that include a modified food starch, a sugar alcohol, or a combination thereof.

The total amount of the purified cannabinoid mixture and/or the ratio of cannabinoid mixture to water soluble agent(s) may be selected based on the desired concentration of cannabinoid compounds and preferences of the consumer. Thus, for example, the composition may have a lower ratio of cannabinoid mixture to water soluble agent when intended for a lower dose of cannabinoids and a higher ratio of cannabinoid mixture to water soluble agent when intended to a higher, recreational dose of cannabinoids. Compositions with a relatively lower ratio of cannabinoid per water soluble agent may be preferred in some cases to allow for more precise control over the amount of cannabinoid added to a beverage or food, and thus more control over the ultimate cannabinoid dosage consumed. In other examples, the consumer may prefer compositions having a higher ratio of cannabinoid per water soluble agent to provide for a higher dosage of cannabinoids when added to a beverage or food.

Yet another exemplary composition herein in the form of a particulate composition, e.g., a powder (which includes agglomerated particles), comprises from about 0.5% to about 20.0% by weight of a purified cannabinoid mixture, from about 0.5% to about 20.0% by weight of a carrier oil, from about 2.5% to about 20.0% by weight of a complex carbohydrate, and optionally from about 10.0% to about 95.0% by weight of a polysaccharide, surfactant, polyol, or combination thereof, wherein the purified cannabinoid mixture comprises at least 80% by weight THC or CBD relative to the total weight of the purified cannabinoid mixture. In another example, the composition in the form of a particulate composition comprises from about 1.0% to about 5.0% by weight of a purified cannabinoid mixture, from about 2.0% to about 10.0% by weight of a carrier oil, from about 70.0% to about 95.0% by weight of at least one of a surfactant, polyol, or polysaccharide, and optionally from about 1.0% to about 10.0% by weight of a complex carbohydrate, wherein the purified cannabinoid mixture comprises at least 80% by weight THC or CBD relative to the total weight of the purified cannabinoid mixture. In yet another example, the particulate composition comprises from about 1.0% to about 30.0% by weight of a purified cannabinoid mixture, from about 1.0% to about 20.0% by weight of a carrier oil, from 0 to about 7.5% by weight of a complex carbohydrate, and from about 80.0% to about 95.0% by weight of a polyol, polysaccharide, or combination thereof, wherein the purified cannabinoid mixture comprises at least 80% by weight THC or CBD relative to the total weight of the purified cannabinoid mixture. Such particulate compositions may comprise, for example, from 0% to about 10.0% by weight water, such as from about 0.5% to about 7.5% by weight, from about 1.0% to about 5.0% by weight, or from about 2.0% to about 4.0% by weight of water, e.g., about 1.0%, about 1.5%, about 2.0%, about 2.5%, about 3.0%, about 3.5%, about 4.0%, about 4.5%, or about 5.0% by weight water.

The compositions herein may be prepared by blending or otherwise combining a hydrophobic component (comprising cannabinoid compounds, e.g., in a purified cannabinoid mixture, and optionally one or more carrier oils and/or one or more antioxidants) with a hydrophilic component (e.g., water and one or more water soluble agents) to form an emulsion. For example, the purified cannabinoid mixture may be combined with water and the water soluble agent(s) to form an oil-in-water (or water-in-oil) emulsion. A paddle mixer, homogenizer, rotor-stator, and/or other suitable mixing device may be used to thoroughly combine the hydrophilic and hydrophobic components with mechanical agitation to produce the emulsion. The components may be combined in any order. In one non-limiting example, the water soluble agent(s) may first be dispersed in water to hydrate or dissolve to water soluble agent(s), and then the purified cannabinoid mixture (and any carrier oil(s)) added to the hydrated water soluble agent(s), e.g., with mixing.

In some examples, mixing/emulsification may include centrifugal force, e.g., using the centrifugal pump of a tri-blender or similar device, to blend dry powders (e.g., water soluble agent(s)) under agitation while metering cannabinoid oil into the powder. A commercial example of a tri-blender device is the Hybrid Powder Mixer produced by Alpha Laval.

Depending on the type, degree, and duration of mixing, the emulsion thus produced may have a $d_{90}$ droplet size (the diameter at which 90% by weight of the oil droplets of the emulsion have a smaller diameter) ranging from

about 100 nm or less (generally described as a nanoemulsion) to 2 μm or greater (generally described as a microemulsion). For example, a paddle mixer may be used to prepare an emulsion having a $d_{90}$ droplet size greater than or equal to 2 μm, e.g., ranging from 2 μm to about 8 μm or from 2 μm to about 5 μm, while homogenization may be used to prepared an emulsion having a smaller $d_{90}$ droplet size of less than 2 μm or less than 500 nm, e.g., ranging from 100 nm to 1.5 μm or 50 nm to 500 nm. The mixing speed during emulsification may range from about 5,000 to 12,000 revolutions per minute (rpm), for example, such as from about 7,000 to 10,000 rpm or from about 6,000 to 8,000 rpm, e.g., a speed of about 5,000 rpm, about 6,000 rpm, about 7,000 rpm, about 7,500 rpm, about 7,800 rpm, about 8,000 rpm, about 8,500 rpm, about 9,000 rpm, about 9,500 rpm, about 10,000 rpm, about 10,500 rpm, about 11,000 rpm, about 11,500 rpm, or about 12,000 rpm. In some examples, the components may be heated during emulsification. For example, emulsification may be performed at a temperature ranging from about 30°C to about 80°C, from about 40°C to about 75°C, from about 30°C to about 50°C, or from about 60°C to about 70°C, e.g., about 40°C, about 45°C, about 50°C, about 55°C, about 60°C, about 65°C, about 70°C, about 75°C, or about 80°C. In some examples, emulsification may be performed for a period of time ranging from about 5 minutes to about 90 minutes, from about 10 minutes to about 60 minutes, from about 15 minutes to about 45 minutes, or from about 30 minutes to about 45 minutes, e.g., about 15 minutes, about 30 minutes, about 45 minutes, or about 60 minutes. In some examples, emulsification may be performed for a period of time ranging from 15 minutes to 45 minutes within a temperature range of 20°C to 80°C, using a mixer that runs at a range of 5,000 rpm to 12,000 rpm.

Homogenization generally refers to the process of incorporating two immiscible liquids to form a homogeneous (or more homogeneous) mixture, e.g., by reducing the droplet size (e.g., oil droplet size) of the dispersed phase. In at least one example, homogenization may be performed at a mixing speed of about 12,000 rpm for 15 to 45 minutes, e.g., about 30 minutes, at a temperature ranging from 60°C to 80°C, e.g., about 70°C. Equipment useful for homogenization includes, for example, a rotor-stator, which spins a rotor at high speed to "cut" the dispersed phase into smaller particles or droplets. A commercial example of a rotor-stator is the T25 ULTRA-TURRAX® produced by IKA. Further, for example, pressure homogenization may be used to apply pressures of 2500-3500 psi (1 psi = 6.89 kPa) to cause droplets (e.g., oil or fat droplets) to "implode" under pressure. A commercial example of a pressure homogenizer is the Crepaco 5 DL produced by APV. Higher pressure homogenizers operating at greater than 10,000 psi (e.g., up to 32,000 psi) also may be used to produce nanoemulsions with a $d_{90}$ droplet size less than 300 nm. A commercial example of a high pressure homogenizer is the M-1 10C Microfluidizer® produced by Microfluidics for homogenization pressures up to 30,000 psi. Further, ultrasonics may be used in homogenization to prepare nanoemulsions with a $d_{90}$ droplet size less than 300 nm. A commercial example of an ultrasonic homogenizer is the BSP-1200 produced by Industrial Sonomechanics. In some examples herein, high pressure homogenization at a pressure up to 32,000 psi may be used to reduce the $d_{90}$ droplet size to less than 800 nm, less than 700 nm, less than 600 nm, or less than 500 nm, e.g., to provide for long-term physical stability suitable for a liquid concentrate to be added to beverages and food products. Exemplary emulsions herein have a $d_{90}$ oil droplet size ranging from about 15 nm to about 50 μm, such as from about 2 μm to about 10 μm, from about 1 μm to about 2.5 μm, from about 250 nm to about 2 μm, from about 500 nm to about 1 μm, from about 100 nm to about 800 nm, from about 100 nm to about 700 nm, from about 200 nm to about 800 nm, from about 200 nm to about 500 nm, or from about 50 nm to about 250 nm. In at least one example, homogenization may be performed at a pressure ranging from about 20,000 psi to about 32,000 psi.

The emulsion may be prepared in two or more steps. For example, a first emulsion may be prepared having an oil droplet size ($d_{90}$ diameter) greater than or equal to 2 μm, such as ranging from about 2 μm to about 10 μm, from about 2 μm to about 6 μm, or from about 4 μm to about 8 μm (e.g., via a rotor stator mixer), and then the droplet size of the first emulsion reduced through further mixing (e.g., via a homogenizer or rotor stator) to produce a second emulsion having a $d_{90}$ oil droplet size less than 2 μm, less than 1 μm, or less than 500 nm. For example, homogenization may reduce the droplet size of the first emulsion to provide for greater stability and/or a more homogeneous emulsion. The first emulsion may be sufficiently stable to allow for the droplet size to be reduced to form the second emulsion. For example, the first emulsion may be stable on the order of hours to weeks, whereas the second emulsion may be stable on the order of months to years. Droplet size reduction may contribute to emulsion stability and/or clarity, may reduce the amount of water soluble agent(s) used, and/or may improve solubility of the emulsion or a composition (e.g., particulate, liquid concentrate, or film) ultimately produced from the emulsion. With respect to clarity of the emulsion, for example, a maximum droplet size smaller than the wavelength of visible light may result in less scattering of light as the wavelengths of light pass through the emulsion. Such emulsions may have a $d_{90}$ droplet size of less than or equal to 800 nm, less than or equal to 700 nm, less than or equal to 600 nm, less than or equal to 500, nm or less than or equal to 400 nm, for example, less than 350 nm, or less than 300 nm.

For subsequent steps of some manufacturing processes herein (not within the scope of the claims), it may be beneficial for the emulsion to have an oil droplet size of less than 2 μm, less than 1 μm, less than 800 nm, less than 500 nm, less than 400 nm, less than 300 nm, less than 200 nm, or less than 100 nm. Such emulsions may be useful as liquid concentrates, e.g., to add to liquids to produce shelf-stable beverages and/or incorporated into food products such as gummy products and energy gels, or may be further processed into particulates or a film coating, as discussed below.

Thus, for example, the hydrophilic and hydrophobic components may be sufficiently blended with water (in one or more steps) to produce an emulsion having a $d_{90}$ droplet size ranging from about 35 nm to about 1.5 μm, such as from about 200 nm to about 1 μm, from about 100 nm to about 500 nm, from about 100 nm to about 250 nm, from about 200 nm to about 700 nm, from about 500 nm to about 1.5 μm, about 500 nm to about 1 μm, or from about 300 nm to about 800 nm. Such emulsions may have an apparent viscosity less than or equal to 700 cP, e.g., less than or equal to 600 cP, or less than or equal to 500 cP. For example, the emulsion may have a viscosity ranging from about 50 cP to about 500 cP, or from about 300 cP to about 400 cP. In some examples, the emulsion may have a viscosity that allows the emulsion to readily flow through a spray nozzle, e.g., during a spray-drying process or film coating process. For example, the viscosity may range from about 100 cP to about 500 cP. In cases in which the emulsion is prepared with heating, the emulsion may be cooled to room temperature prior to drying.

The emulsions herein (not within the scope of the claims) may be provided as liquid concentrate compositions, e.g., via high pressure homogenization as discussed above. The liquid concentrate may be suitable for adding to beverages and food products, for example. Such beverages include, but are not limited to, juices, smoothies, alcoholic beverages and other fermented liquids, carbonated liquids, and sports and energy drinks. The liquid concentrate may have sufficient stability to stay in emulsion form over long periods of time. For example, the liquid concentrate may be an emulsion with a $d_{90}$ oil droplet size less than or equal to about 800 nm, or less than or equal to about 500 nm, e.g., from about 50 nm to about 800 nm, from about 50 nm to about 500, or from about 100 nm to about 400 nm. The liquid concentrate may be physically stable (remaining in emulsion form) for a year or more, such that the liquid concentrate does not coalesce, ring (e.g., separate into layers), or settle in the beverage or food product over time.

In some examples, the liquid concentrate may comprise a purified cannabinoid mixture (e.g., comprising CBD, THC, and/or one or more other cannabinoid compounds) and at least one carrier oil, wherein the total oil load of the emulsion is up to 60% by weight, such as from about 0.05% to about 50.0%, from about 5.0% to about 50.0%, from about 1.0% to about 40.0%, from about 10.0% to about 30.0%, from about 25.0% to about 45.0%, from about 45.0% to about 60.0%, from about 30.0% to about 50.0%, or from about 20.0% to about 35.0% by weight, with respect to the total weight of the liquid concentrate. Even at such levels, the liquid concentrate may be capable of producing a substantially clear emulsion when mixed into a clear beverage, as opposed to giving the beverage a cloudy appearance.

In some examples, the weight ratio of carrier oil to purified cannabinoid mixture in the liquid concentrate ranges from about 1:4 to about 2:1, from about 3:4 to about 4:3, from about 1:2 to about 1:1, or from about 1:2 to about 4:3. For example, the composition may comprise from about 20.0% to about 30.0% by weight of a carrier oil and from about 20.0% to about 30.0% by weight of a purified cannabinoid mixture at a weight ratio of carrier oil to purified cannabinoid mixture from about 1:4 to about 2:1, from about 3:4 to about 4:3, from about 1:2 to about 1:1, or from about 1:2 to about 4:3. In at least one example, the liquid concentrate comprises at least one carrier oil chosen from MCT oil, coconut oil, hemp oil, or a mixture thereof; and the purified cannabinoid mixture comprises CBD, THC, or a combination thereof. Additionally or alternatively, the water soluble agent(s) of the liquid concentrate may have a weight ratio of water soluble agent(s) to oil (the hydrophobic component comprising the carrier oil(s) and the purified cannabinoid mixture) ranging from about 5:1 to about 1:5, from about 4:1 to about 1:4, from about 3:1 to about 1:3, from about 2:1 to about 1:2, e.g., a ratio of about 1:1. In at least one example, the liquid concentrate comprises one or more water soluble agents chosen from modified food starch, quillaja extract, gum arabic, or a combination thereof. In at least one example, the liquid concentrate does not comprise sorbitan, a sorbitan ester, a polysorbate, or maltodextrin.

In some examples, the cannabinoid content of the liquid concentrate may range from about 1.0 mg to about 50.0 mg per serving, such as from about 1.0 mg to about 25.0 mg per serving or from about 5.0 mg to about 10.0 mg per serving, wherein a serving corresponds to a volume of about 4 oz. to about 16 oz. liquid, such as about 12 oz. (1 oz. = 0.118 liter). In some examples, the composition comprises a beverage having a cannabinoid concentration ranging from about 1.0 mg to about 25.0 mg per serving, such as from about 2.5 mg to about 20.0 mg per serving, from about 5.0 mg to about 10.0 mg per serving, wherein a serving corresponds to a fluid volume of about 4 oz., about 6 oz., about 8 oz., about 10 oz., about 12 oz., about 14 oz., or about 16 oz. The beverage may have a neutral or acidic pH, such as a pH less than about 7.0, less than about 6.0, less than about 5.0, or less than about 4.0. In some examples, the pH of the beverage ranges from about 3.5 to about 7.0, from about 4.0 to about 6.0, or from about 4.5 to about 5.5. The beverage may be thermally processed for extended microbial shelf life. In some examples, the beverage is shelf stable, e.g., having a shelf life of at least 90 days at 5°C and/or a shelf life of at least 1 year at 20°C.

Further, for example, the emulsion may comprise a purified cannabinoid mixture in an amount ranging from about 1% to about 20% by weight, based on the total weight of the emulsion, such as from about 5% to about 10%, from about 15% to about 20%, from about 1% to about 5%, from about 8% to about 12%, from about 10% to about 14%, or from about 12% to about 17% by weight. Additionally or alternatively, the emulsion may comprise from about 40% to about 75% by weight water, such as from about 50% to about 60%, from about 55% to about 75%, from about 45% to about 60%, or from about 55% to about 65% by weight water, based on the total weight of the emulsion. The emulsion may comprise at least one water soluble agent, such as a complex carbohydrate, in an amount ranging from about 10% to about 60% by weight, from about 20% by weight to about 50% by weight, or from about 40% to about 50% by weight,

with respect to the total weight of the emulsion. Without intending to be bound by theory, it is believed that carbohydrates protect the cannabinoids from oxidation (e.g., slowing the rate of oxygen contact with cannabinoids). In some examples, the emulsion may further comprise an antioxidant, such as rosemary extract, to provide additional oxidative stability.

The emulsion may have a viscosity suitable for application to a substrate, e.g., via spraying process. For example, the viscosity of the emulsion may range from about 100 cP to about 400 cP, such as from about 150 cP to about 300 cP, from about 200 cP to about 350 cP, from about 300 cP to about 400 cP, from about 250 cP to about 350 cP, or from about 100 cP to about 200 cP.

Further, according to some aspects of the present disclosure, the emulsion (including, e.g., any of the emulsions described above or elsewhere herein) may be dried into particles or otherwise further processed to form particles. For example, the emulsion may be spray dried by passing the emulsion through a spray nozzle to at least partially or completely atomize or vaporize the emulsion into aerosols or small droplets. The aerosols thus produced may be mixed with warm or hot, dry air (or other inert gas such as helium or nitrogen) to rapidly remove moisture and form a particulate composition, such as a powder, via evaporative cooling in the drying chamber. The temperature of the air may range from about 30°C to about 200°C, such as from about 30°C to about 150°C, from about 35°C to about 75°C, from about 50°C to about 150°C, from about 45°C to about 90°C, or from about 80°C to about 120°C. For example, spray drying may remove 50% or more of the moisture to leave a particulate composition with 10% or less water moisture by weight, such as from about 0.1% to 10% by weight, from about 0.5% to about 7.5% by weight, from about 1.0% to about 8.0% by weight, from about 1.0% to about 5.0% by weight, or from about 1.5% to about 3.0% by weight water moisture. The powder then may be separated from the drying air, e.g., based on density or other physical or chemical characteristics, and the powder collected. Spray-drying may be performed as a batch process or a continuous process. The powder may be a flowable powder (e.g., flowable granules) or a compressible powder.

In some cases, it may be desirable for the heated air to use lower drying temperatures and/or shorter drying times to promote greater product stability by reducing oxidative stress and thermal degradation of the components of various actives/bioactives in the composition, including cannabinoid compounds. Lower drying temperatures also may be compatible with a wider range of ingredients, which can be useful for preparing formulations with the appropriate level of water solubility. For example, certain water soluble agents such as sorbitol tend to form a sticky material with poor water solubility at the higher temperatures typical of many spray drying processes.

Accordingly, in some aspects of the present disclosure, spray-drying is performed at a temperature less than or equal to about 80°C, less than or equal to about 70°C, less than or equal to about 60°C, less than or equal to about 50°C, or less than or equal to about 40°C, e.g., a temperature ranging from about 25°C to about 80°C, from about 30°C to about 60°C, from about 25°C to about 50°C, from about 45°C to about 75°C, or from about 40°C to about 55°C. Additionally or alternatively, the residence time in the spray drying chamber may be less than or equal to 1 hour, less than or equal to 45 minutes, less than or equal to 30 minutes, less than or equal to 20 minutes, less than or equal to 15 minutes, less than or equal to 5 minutes, or less than or equal to 2 minutes, such as from about 5 minutes to about 45 minutes, or from about 20 minutes to about 30 minutes.

In some aspects of the present disclosure, the spray drying may be electrostatic spray drying, wherein the components of the emulsion have differing polarities. For example, during electrostatic spray drying, molecules with greater polarity (e.g., water, water soluble agents and other hydrophilic components) repel each other and typically migrate to the outer portion of the droplets, while the less polar molecules (e.g., cannabinoids, carrier oils, and other hydrophobic components) remain towards the center of the droplets. This migration allows for encapsulation of the cannabinoids by the water soluble agent and evaporation of the water at lower temperatures.

As mentioned above, the powder may be a flowable powder. Providing the composition as a flowable powder may have certain advantages in packaging and/or handling. For example, flowable powders can facilitate some filling processes, such as VFFS. Flowable powders also may provide for greater control over dosing. For example, consumers and vendors may find it easier to measure and scoop a flowable powder for addition to a beverage or food product such as coffee, or a freshly-juiced fruit or vegetable drink, to achieve the desired dose of cannabinoids. The Hausner ratio provides an indication of the flowability of a powder or granular material, and is calculated by dividing the tapped bulk density of the material ($\rho_{tapped}$) by its freely settled bulk density ($\rho_{bulk}$):

$$Hausner\ Ratio = \left(\frac{\rho_{tapped}}{\rho_{bulk}}\right) \qquad \text{Equation 1}$$

The tapped bulk density refers to the density of the powder after a specified compaction process, such as vibration of a container housing a specified volume of powder. The Hausner ratio depends on the technique used to measure density. The freshly settled bulk density can be measured by introducing a particulate sample into a 50-mL (50 $cm^3$) graduated cylinder, measuring its mass without compacting the sample, and dividing the mass by the 50 mL volume. The tapped density of the same sample can be measured by mechanically tapping the side of the graduated cylinder until the

particulate level is fully settled (the measured volume comes to a steady value) to effectively compact the sample. The volume of the tapped sample is then measured, and the sample mass divided by the compacted volume. A Hausner ratio at or close to 1 describes materials with high flowability, whereas higher ratios around 1.3 and greater indicate poor flowability and higher compressibility. See Table 1 below. The compressibility index may be calculated as follows:

$$Compressibility\ Index = 100\ \times \left(\frac{\rho_{tapped} - \rho_{bulk}}{\rho_{tapped}}\right) \qquad \text{Equation 2}$$

Table 1

| Flow character | Hausner Ratio | Compressibility Index |
|---|---|---|
| Excellent/very free flow | 1.00-1.11 | ≤ 10 |
| Good/free flow | 1.12-1.18 | 11-15 |
| Fair | 1.19-1.25 | 16-20 |
| Passable | 1.26-1.34 | 21-25 |
| Poor/cohesive | 1.35-1.45 | 26-31 |
| Very poor/very cohesive | 1.46-1.59 | 32-37 |
| Very, very poor/approx. non-flow | > 1.60 | > 38 |

For example, the mass of a freely settled particulate sample may be measured at 30.2 g, providing a $\rho_{bulk}$ of 0.60 g/cm$^3$, and after tapping, the volume may be measured at 45 mL, providing a $\rho_{tapped}$ of 0.67 g/cm$^3$. The Hausner ratio then would be 1.11, corresponding to high or "excellent" flowability. In some examples herein, the composition may be or comprise a powder with a Hausner ratio less than or equal to 1.25, less than or equal to 1.20, or less than or equal to 1.10; and/or a compressibility index less than or equal to 20, less than or equal to 15, or less than or equal to 10.

According to some aspects of the present disclosure (not within the scope of the claims), the composition is or comprises a flowable powder having a cannabinoid concentration ranging from about 1.0% to about 25.0% by weight. In some examples, the powder comprises one or more water soluble agents chosen from food modified starch, sorbitol, maltodextrin, gum arabic, quillaja extract, or a combination thereof. In at least one example, the powder is flowable and does not comprise maltodextrin, sorbitan, sorbitan ester, or polysorbate. The powder may be a flowable powder, e.g., having a Hausner ratio less than 1.25 and a compressibility index less than 20%. In some examples, the flowable powder is prepared from an emulsion comprising from about 1.0% to about 20.0% by weight cannabinoids, at least one carrier oil, water, and one or more water soluble agents at a temperature ranging from about 30°C to about 150°C, such as from about 30°C to about 60°C.

The compositions herein are in the form of agglomerated particles. Agglomeration may help to increase flowability and/or solubility of the composition, e.g., by providing the composition with a lower bulk density and/or larger surface area having many voids and cavities, somewhat similar to forming snow from sleet. The agglomerated particles may provide for a more homogeneous composition and free-flowing structure. Agglomeration may include applying pressure or shear forces to the particles to form a porous structure. This porous or honeycomb-type structure may promote or otherwise control how water permeates the composition in order to increase and control the water solubility of the composition.

Agglomeration may be performed using a fluidized bed. In this process, an emulsion is metered into the processing chamber, e.g., in a semi-continuous fashion, wherein the liquid mixes with pressurized air to atomize the liquid emulsion into a fine mist. The atomized emulsion droplets then build on particles pre-charged into the fluidized bed chamber to build up particle size.

Any of the emulsions disclosed herein may be used in agglomeration. For example, the emulsion may have a $d_{90}$ droplet size less than 2 $\mu$m, e.g., less than or equal to 500 nm, or a $d_{90}$ droplet size greater than 2 $\mu$m, e.g., ranging from 2 $\mu$m to about 8 $\mu$m. Thus, for example, the emulsion may be prepared with a paddle mixer and/or a rotor-stator as discussed above, among other suitable mixing techniques. The emulsion used to prepare agglomerated particles typically comprises a purified cannabinoid mixture, at least one carrier oil, at least one water soluble agent, and water. In some examples, the water soluble agent comprises a modified food starch, sorbitol, maltodextrin, quillaja extract, or a combination thereof. Further, for example, the carrier oil may comprise MCT oil, coconut oil, hemp oil, or a mixture thereof; and the purified cannabinoid mixture may comprise CBD, THC, and/or one or more other cannabinoid compounds.

In at least one example, the emulsion is prepared by mixing the components to produce a $d_{90}$ oil droplet size ranging from about 2 μm to about 8 μm. Similar to the film coating processes and other drying processes discussed above, the emulsion may have a relatively low viscosity suitable for atomization. For example, the viscosity of the emulsion may range from about 100 cP to about 400 cP, such as from about 100 cP to about 300 cP, or from about 150 cP to about 250 cP.

The particles pre-charged into the fluidized bed chamber may comprise a carbohydrate, which may be the same or different from a carbohydrate used as a water soluble agent in the emulsion. In some examples, the pre-charged particles comprise one or more water soluble agents as described above. For example, the pre-charged particles may comprise a carbohydrate, such as a complex carbohydrate or a sugar, a polyol, such as a sugar alcohol; a protein powder; a fiber (e.g., soluble fiber and/or insoluble fiber); or a combination thereof. Water soluble materials suitable for the agglomerated particles herein include, but are not limited to, starches, including modified food starches, gum arabic, and quillaja extract; sorbitol, maltitol, and other sugar alcohols, maltodextrin, dextrose, dextrin, corn syrup solids, soluble fibers, and combinations thereof. Similar to the discussion above regarding spray drying, using lower temperatures may help to avoid degradation of various components and/or allow for a wider range of water soluble agents and other ingredients to control the water solubility of the composition.

The residence time during agglomeration in the fluidized bed may be chosen based on the desired size of the agglomerated particles. The particulate compositions herein optionally may classified, e.g., in a screening process. For example, the particulate material may be passed through multiple sieves with specific mesh sizes. Thus, the particle size distribution of a given material may be expressed in terms of the percentage of the particles (by weight) in the material having a size that is greater or less than the screen size of the mesh. For example, the particle size of the particulate compositions herein may be measured by placing a 500 gram sample on top of a sieve stack arranged with the largest screen size on top to the smallest screen size on the bottom (e.g., mesh sizes 18, 20, 30, 40, 50, and 60). The sieve stack is placed on an orbital shaker table and shaken for about 5 minutes, after which the mass of material collected on each screen is measured. The following table lists mesh sizes and the corresponding size in microns (μm).

Table 2

| Mesh size | Opening |
|---|---|
| 18 | 1000 μm |
| 20 | 841 μm |
| 25 | 707 μm |
| 30 | 595 μm |
| 35 | 500 μm |
| 40 | 420 μm |
| 50 | 297 μm |
| 60 | 250 μm |
| 70 | 210 μm |
| 100 | 149 μm |
| 140 | 105 μm |
| 200 | 74 μm |

According to some aspects of the present disclosure, at least 40%, at least 50%, at least 60%, at least 70%, at least 80%, at least 90%, or at least 95% by weight of the agglomerated particles may have a particle size between about 150 μm and about 800 μm. Additionally or alternatively, at least 40%, at least 50%, at least 60%, at least 70%, at least 80%, at least 90%, or at least 95% by weight of the agglomerated particles may have a particle size between about 250 μm and about 800 μm, between about 150 μm and about 600 μm between about 300 μm and about 600 μm, or between about 250 μm and about 600 μm. Smaller sized particles (e.g., particles less than about 150 μm, less than about 100 μm, or less than about 75 μm) and/or larger sized particles (e.g., particles greater than about 600 μm, greater than about 710 μm, or greater than about 850 μm) optionally may be removed prior to packaging.

According to some aspects of the present disclosure, the agglomeration process may be performed under steady-state conditions, e.g., wherein the emulsion continues to be fed into the fluidized bed chamber at approximately the same rate that the agglomerated particles are removed. In some examples, a particulate composition, such as the

spray-dried particles herein, may be agglomerated to increase solubility. For example, a particulate composition may be prepared by spray drying an emulsion, and then the spray-dried particles may be combined with a liquid to agglomerate the particles.

Following agglomeration, the composition may have a bulk density less than about 0.6 g/cm$^3$, e.g., less than 0.5 g/cm$^3$ or less than 0.4 g/cm$^3$. According to some aspects of the present disclosure, for example, the particulate composition may have a bulk density ranging from about 0.2 g/cm$^3$ to about 0.8 g/cm$^3$, from about 0.3 g/ cm$^3$ to about 0.7 g/cm$^3$, from about 0.4 g/ cm$^3$ to about 0.7 g/cm$^3$, from about 0.3 g/cm$^3$ to about 0.5 g/cm$^3$, or from about 0.3 g/cm$^3$ to 0.4 g/cm$^3$, e.g., a bulk density of about 0.2 g/cm$^3$, 0.25 g/cm$^3$, 0.3 g/cm$^3$, 0.35 g/cm$^3$, 0.4 g/cm$^3$, 0.45 g/cm$^3$, 0.5 g/cm$^3$, 0.55 g/cm$^3$, 0.6 g/cm$^3$, 0.65 g/cm$^3$, or 0.7 g/cm$^3$.

In some examples, the composition of agglomerated particles comprises from about 0.05% to about 35.0% by weight of the purified cannabinoid mixture, with respect to the total weight of the composition, such as from about 0.1% to about 25.0% by weight. Additionally or alternatively, in some examples, the composition comprises from about 25.0% to about 95.0% by weight of the at least one water soluble agent, with respect to the total weight of the composition, such as from about 35.0% to about 95.0% by weight, from about 40.0% to about 95.0% by weight, from about 50.0% to about 95.0% by weight, or from about 60.0% to about 95.0% by weight. Further, for example, the composition may comprise from about 3.0% by weight to about 30.0% by weight of at least one carrier oil, with respect to the total weight of the composition, such as from about 3.0% to about 20.0% by weight, or from about 3.0% to about 10.0% by weight. The purified cannabinoid mixture and the at least one carrier oil may form a hydrophobic component of the composition, wherein the weight ratio of the water soluble agent(s) to the hydrophobic component ranges from about 1:5 to about 5:1, such as from about 1:4 to about 4:1, from about 1:3 to about 3:1, from about 1:2 to about 2:1, or about 1:1.

Various flavoring agents, coloring agents, antioxidants/stabilizing agents, as well as any other suitable nutritional additives or natural preservatives may be added at various steps of the manufacturing methods herein.

The particulate compositions herein may be soluble in cold water, e.g., water at a temperature of about 20°C or less. That is, the composition particles may dissolve in the water within 30 seconds, within a minute, or within a few minutes with gentle mixing to form a clear or translucent/somewhat cloudy solution, wherein the solution remains stable with minimal or no particles undissolved or settling out of solution. According to some aspects of the present disclosure, the composition may be completely soluble in water at a temperature of 20°C or greater, and at least partially soluble in water at a temperature less than 20°C, e.g., ranging from 5°C to 20°C. Further, for example, the composition may be completely soluble in water at a temperature of 10°C or higher, and at least partially soluble in water having a temperature ranging from 5°C to 10°C. For example, the compositions herein may be characterized as having excellent, good, or fair solubility in water at a temperature ranging from 5°C to 20°C, wherein a solubility time of less than 20 seconds = excellent solubility, 20-30 seconds = good solubility, 1-3 minutes = fair solubility, 3-5 minutes = poor solubility, and greater than 5 minutes = insoluble. Solubility of the particulate compositions can be measured by adding a 400 mg sample to 240 mL (8 oz.) of water at the specified temperature with continuous mixing at about 300 rpm. In an exemplary procedure, water added into 250 ml glass beaker set on a magnetic stirrer, and a magnetic stir bar (3/4" long) is added and set to about 300 rpm to create a slight vortex. A 400 mg sample of the test power is poured into the water, and the time for all particles to dissolve is measured. In some examples, the compositions herein may dissolve in 240 ml of water at a temperature less than or equal to 20°C within 30 seconds, within 25 seconds, within 20 seconds, within 15 seconds, within 10 seconds, or within 5 seconds. The particulate compositions herein may be about the same or more soluble than sucrose (table sugar) under the same conditions.

The ratios of water soluble agent(s), oil(s), and/or water relative to one another may be selected to provide particles with the desired solubility characteristics and cannabinoid dosages. For example, if the ratio of oil(s) (hydrophobic component) to water soluble agent(s) (hydrophilic component) is too high, the emulsions may result in particles with insufficient solubility in cold water and/or may form an oil-slick at the surface of the water. In some examples, the mass ratio of water soluble agent to oil (cannabinoid mixture or cannabinoid mixture with carrier oil) may range from about 1:3 to about 3:1, e.g., a mass ratio of about 0.5 (i.e., 1:2), about 0.7, about 0.9, about 1 (i.e., 1:1), about 1.1, about 1.3, about 1.5, about 1.7, or about 2.0 (i.e., 2:1). In at least one example, the ratios of water soluble agent (e.g., modified food starch), purified cannabinoid mixture/carrier oil, and water in the emulsion are 1:1:2.

In some examples that comprise one or more carrier oils, the mass ratio of carrier oil(s) to cannabinoid mixture may range from about 1:3 to about 3:1, e.g., a mass ratio of about 0.5 (i.e., 1:2), about 0.7, about 0.9, about 1 (i.e., 1:1), about 1.1, about 1.3, about 1.5, about 1.7, or about 2.0 (i.e., 2:1).

The compositions herein may be further processed and/or packaged, depending on the final uses of the product. For example, the composition in particulate form (e.g., a powder including agglomerated particles) may be packaged in individual sachets or packets for single use by a consumer, and/or may be packaged into larger canisters or other containers, including in bulk, for repeated use by a consumer or multiple consumers. The packets or other containers may be marked with the corresponding dosage of THC, CBD, and/or other cannabinoids. Exemplary doses range from "micro doses" of about 2.5 mg/serving or less (e.g., about 0.5 mg/serving or less) to larger doses ranging from about 5 mg/serving to about 10 mg/serving, e.g., for recreational use. The present disclosure contemplates doses higher than

10 mg/serving, e.g., for medicinal use, such as doses of 50 mg/serving or greater. Exemplary dosages may range from about 0.5 mg/serving to about 50.0 mg/serving, such as from about 1.0 mg/serving to about 2.5 mg/serving, from about 1.0 mg/serving to about 5.0 mg/serving, from about 10.0 mg/serving to about 15.0 mg/serving, from about 10.0 mg/serving to about 30.0 mg/serving, from about 5.0 mg/serving to about 25.0 mg/serving, from about 35.0 mg/serving to about 50.0 mg/serving, or from about 20.0 mg/serving to about 40.0 mg/serving. A serving size for beverages and food products typically ranges from about 4 oz. to about 16 oz. Serving sizes for food products typically range from about 2 g to about 50 g, such as about 10 g for a serving of popcorn, about 30 g to 50 g for a serving of nuts, dried fruit, or trail mix, etc. The dosage of cannabinoid(s) of the composition may be chosen to achieve any of the benefits associated with cannabinoid compounds listed above (e.g., decreasing pain and/or nausea, reducing stress, promoting sleep, boosting energy, etc.). According to some aspects of the present disclosure, the composition (particulate composition, such as a powder) may be added to a food or beverage to provide a dose of cannabinoid compounds ranging from about 0.1 mg to about 50.0 mg or more, such as from about 0.5 mg to about 30.0 mg, from about 1.5 mg to about 2.5 mg, from about 5.0 mg to about 25.0 mg, from about 10.0 mg to about 30.0 mg, from about 15.0 mg to about 20.0 mg, from about 30.0 mg to about 45.0 mg, or from about 40.0 mg to about 50.0 mg.

The compositions herein may be added to a wide variety of beverages and foods without significantly affecting the taste, smell, and/or viscosity (including e.g., mouth feel or texture) of the beverage or food. Such beverages/foods include, without limitation, plain or flavored water (which also may be carbonated, for example, or frozen into ice cubes), tea, coffee, soda, fruit juices and smoothies, sport drinks, energy drinks, energy gels, energy bars, fermented beverages (e.g., alcohol, kombucha), milk and other dairy-based foods and beverages (including yogurt and ice cream), non-dairy substitutes, sauces, soups, snacks (e.g., nuts, dried fruit, trail mix, pretzels, popcorn), and desserts (e.g., brownies, cookies, cakes, muffins, sorbet, popsicles, hard candies, etc.), among many other examples. In some examples, adding or incorporating the compositions herein into a liquid, such as a beverage, does not create an oily film or provide the liquid with an oil slick-like appearance. In some examples herein, the composition may be used as a supplement, e.g., for recreational or medical use.

The compositions herein may be low- to zero-calorie, such that the addition to beverages and foods does not substantially add to the sugar content or amount of calories. The compositions herein may be free from one or more of common food allergens (milk, eggs, peanuts, tree nuts, fish, shellfish, soy and wheat). For example, the compositions herein may be vegan, nut-free, dairy-free, and/or gluten-free. The compositions herein may be free from genetically-modified non-organisms (non-GMO), may comprise organic ingredients, and/or may be clean label.

It is understood that the foregoing compositions are exemplary only, and additional compositions are encompassed by the disclosure herein.

## EXAMPLES

The following examples are intended to illustrate the present disclosure without, however, being limiting in nature. It is understood that the present disclosure encompasses additional embodiments consistent with the foregoing description and following examples.

### Example 1 (reference)

A sample composition (Composition A) was prepared by combining 25% by weight canola oil as proxy for cannabinoid extract/carrier oil, 25% by weight modified food starch, and 50% by weight water. The ingredients were combined and emulsified in a high speed mixer at 5000 rpm for two minutes and then homogenized at 7000 rpm for 20 minutes. The resulting emulsion had a mean droplet size of 1.705 $\mu$m, a median particle size of 1.569 $\mu$m, and 82.9% of the particles were above 1 $\mu$m. The emulsified mixture was subsequently pressure homogenized at 5000 psi in 2 passes. After the pressure homogenization, the mean droplet size was decreased to 0.573 $\mu$m with a median droplet size 0.459 $\mu$m, and only 16.7% of the particles were above 1 $\mu$m. Thereafter, the mixture was spray dried to remove moisture, resulting in a particulate or granular composition with about 4% water.

### Example 2 (reference)

Compositions B1 and B2 were prepared from the ingredients listed in Table 3. The cannabinoid oil was a purified distillate comprising either 85% by weight of THC (Composition B1) or 85% by weight of CBD (Composition B2). Each composition was first prepared as an emulsion (chemical composition % wt. (wet)) and then dried into a powder (chemical composition % wt. (dry)).

Table 3

| | Compositions B1 and B2 | |
|---|---|---|
| | % wt. (wet) | % wt. (dry) |
| Modified food starch | 2.50 | 5.88 |
| 18 DE maltodextrin | 45.00 | 84.74 |
| Fractionated coconut oil | 1.24 | 2.93 |
| Cannabinoid oil distillate | 1.25 | 2.94 |
| Rosemary extract | 0.01 | 0.01 |
| Water | 50.00 | 3.50 |
| Total | 100.00 | 100.00 |

To prepare each composition, the water was heated to 60°C in a 250-mL beaker and the starch added with gentle stirring. The maltodextrin was then added with gentle stirring. Separately, the coconut oil was heated to 80°C in a 50-mL beaker and the purified cannabinoid oil slowly added followed by the rosemary extract to form an oil mixture. The oil mixture was then gradually metered into the starch/maltodextrin solution under stirring to form an emulsion. The emulsion was homogenized at 10,000 rpm for 10 minutes at 70°C to reduce the emulsion droplet size to between 2 μm and 10 μm.

The homogenized emulsion was dried on Teflon sheets in a dehydrator at a temperature of about 57°C (135°F) for 3 days, forming thin films. The dried films were broken up into pieces and dried under vacuum. The dried pieces were then granulated into a powder, Composition B1 having a THC content of 2.5% by weight, and Composition B2 having a CBD content of 2.5% by weight.

Example 3 (reference)

Composition C was prepared as an emulsion and then granulated powder from the ingredients shown in Table 4. The cannabinoid oil was a purified distillate comprising 85% by weight of CBD.

Table 4

| | Composition C % wt. (dry) |
|---|---|
| Modified food starch | 0.40 |
| Sorbitol | 90.28 |
| Polysorbate 80 | 0.94 |
| Glycerol monostearate | 0.13 |
| Fractionated coconut oil | 5.30 |
| Cannabinoid oil distillate (85% wt. CBD) | 2.94 |
| Rosemary extract | 0.01 |
| Total | 100.00 |

To prepare Composition C, water was heated to 60°C in a 250-mL beaker and the starch added with gentle stirring. The sorbitol, polysorbate 80, and glycerol monostearate were then added to the hydrated starch solution. Separately, the coconut oil was heated to 80°C in a 50-mL beaker and the purified cannabinoid oil slowly added followed by the rosemary extract to form an oil mixture. The oil mixture was then gradually metered into the starch/maltodextrin solution under stirring to form an emulsion. The emulsion was homogenized at 10,000 rpm for 10 minutes at 70°C and then dried and ground as described in Example 2 to produce a fine powder having a CBD content of 2.5% by weight.

Example 4 (reference)

Compositions D1, E, and F were prepared from the ingredients listed in Table 5 below with varying carrier oil:CBD and starch:oil ratios summarized in Table 6. Each composition was prepared as an emulsion and then dried

and ground into a powder according to the procedure of Example 2, but using canola oil in place of coconut oil, and CBD isolate (>99.5% purity) in place of the purified cannabinoid distillate.

Table 5

| | Composition D1 | | Composition E | | Composition F | |
|---|---|---|---|---|---|---|
| | G | % wt. (wet) | g | % wt. (wet) | g | % wt. (wet) |
| Modified food starch | 77.5 | 38.75 | 25 | 12.50 | 30 | 15.00 |
| 18 DE maltodextrin | 15 | 7.50 | 25 | 12.50 | 10 | 5.00 |
| Canola oil | 5 | 2.50 | 25 | 12.50 | 20 | 10.00 |
| CBD | 2.5 | 1.25 | 25 | 12.50 | 40 | 20.00 |
| Water | 100 | 50.00 | 100 | 50.00 | 100 | 50.00 |
| Total | 200 | 100.00 | 200 | 100.00 | 200 | 100.00 |

Table 6

| | Composition D1 | Composition E | Composition F |
|---|---|---|---|
| Carrier oil:CBD | 2:1 | 1:1 | 0.5:1 |
| Starch: oil | 2:1 | 2:1 | 2:1 |

It was found that a 2:1 starch:oil ratio resulted in the oil being well dispersed in the hydrophilic starch matrix. The final CBD content of the particulate compositions was 2.5% (Composition D), 25% (Composition E), and 40% (Composition F), illustrating that the cannabinoid concentration can be controlled by adjusting the ratio of carrier oil to cannabinoid(s).

Example 5 (reference)

Composition G was prepared from the ingredients listed in Table 7 as a model system using quillaja extract in place of starch for the hydrophilic component, and canola oil as the hydrophobic component.

Table 7

| | Composition G | |
|---|---|---|
| | g | % wt. (wet) |
| 18 DE maltodextrin | 10 | 5.00 |
| Quillaja extract | 30 | 15.00 |
| Canola oil | 60 | 30 |
| Water | 100 | 50.00 |
| Total | 200 | 100.00 |

To prepare Composition G (model system), the water was heated to 60°C in a 250-mL beaker and the quillaja extract added with gentle stirring. The maltodextrin was then added with gentle stirring. The canola oil was heated to 80°C and then gradually metered into the quillaja/maltodextrin solution under stirring to form an emulsion. The emulsion of Composition G was observed to have a lower viscosity than the emulsions of Compositions B1, B2, and D-F using modified food starch. The emulsion was then homogenized at 7,800 rpm for 10 minutes at 70°C to reduce the emulsion droplet size to between 2 μm and 10 μm. The homogenized emulsion was dried on Teflon sheets in a dehydrator at a temperature of about 57°C (135°F) for 3 days, forming thin films. The dried films were broken up into pieces and dried under vacuum. The dried pieces were then granulated into a powder. The quillaja extract was observed to have fair to good cold water solubility.

Example 6 (reference)

For comparison to the "benchtop" drying process of Examples 2-5, Composition D2 was prepared with the same chemical composition of Composition D1 (see Table 5 above) and according to the same procedure, but drying the emulsion by spray-drying rather than using a dehydrator followed by granulation. A pilot scale spray dryer was used at an air temperature of 150°F-180°F at the inlet and 88°F-105°F at the outlet, and a product flow rate of approximately 1 gallon/hr. Air was used as the carrier gas.

The particle size distribution of the resulting powder of Composition D2 was measured by laser diffraction with a Beckman Coulter LS Particle Size Analyzer. Results are shown in Table 8.

Table 8

| | |
|---|---|
| $d_{10}$ | 30.01 μm |
| $d_{25}$ | 71.07 μm |
| $d_{50}$ | 136.0 μm |
| $d_{75}$ | 237.6 μm |
| $d_{90}$ | 420.1 μm |
| average diameter | 187.6 μm |
| | |
| < 10 μm | 2.14% |
| < 25 μm | 7.94% |
| < 44 μm | 15.3% |

The size distribution may be described as the cumulative percentage by weight of particles having a given equivalent spherical diameter. The $d_{10}$ value is the diameter within the distribution at which 10% by weight of the particles have a smaller diameter. Similarly, the $d_{50}$ value is the mean diameter, i.e., the diameter at which 50% by weight of the particles have a smaller diameter. As shown in Table 8, the $d_{50}$ diameter was measured at 136.0 μm, and the $d_{50}$ diameter was measured at 420.1 μm. The end product has good cold water solubility characteristics.

Example 7 (reference)

Water solubility testing was performed for Compositions C, D1, D2, and E described in Examples 3, 4, and 6. Each composition was tested at temperatures of 22°C (tap water) and 10°C (iced tap water). For each test, 240 mL of water at the target temperature (22°C or 10°C) was added to a beaker and a magnetic stirrer used with medium agitation, creating a slight vortex. A 10 mg sample of the composition to be tested was added over a 1-2 second time period and allowed to mix. The time in seconds until no particulates were visually discernable (either suspended in solution or settled out) was recorded as the solubility time. The solubility of table sugar (i.e., granulated sucrose) was also tested according to the same procedure for comparison. Results are shown in Table 9.

Table 9

| Composition | Solubility time (sec) | |
|---|---|---|
| | 22°C | 10°C |
| C | 9 | < 30 |
| D1 | 22 | 60 |
| D2 | < 28 | < 40 |
| E | < 45 | < 30 |
| Table sugar | 22 | < 60 |

The following categories were used to assess solubility characteristics: < 30 sec = excellent to good solubility, 1-3 min = fair solubility, 3-5 min = poor solubility, and > 5 min = insoluble. By this measure, all compositions were found to have good or excellent cold water solubility. All of compositions C, D1, D2, and E were found to be at least as soluble as table sugar, and more soluble in the case of Composition C (using a combination of modified food starch, sorbitol, polysorbate 80, and glycerol monostearate in the hydrophilic component).

As discussed above, Compositions D1 and D2 had the same chemical composition, with Composition D1 dried according to the "benchtop" dehydration process, and Composition D2 being spray-dried.

Example 8 (not within the scope of the claims)

A cannabinoid liquid concentrate of about 25% by weight CBD was prepared according to Table 10 below as follows. Liquid quillaja extract was combined with water to produce an aqueous solution. Separately, the MCT oil and CBD isolate were combined and heated until the CBD completely dissolved. The aqueous and oil components were combined in a high shear mixer, and then homogenized at greater than 20,000 psi. The resulting liquid concentrate emulsion (nanoemulsion) had a $d_{90}$ droplet size less than 500 nm.

Table 10

| Ingredients | % wt. |
|---|---|
| Quillaja extract | 36.0% |
| MCT oil | 25.7% |
| CBD isolate | 23.4% |
| Water | 15.0% |
| Sodium benzoate | * |
| Potassium sorbate | * |
| Citric acid | * |
| Total | 100.0% |
| *For food preservation | |

Example 9 (not within the scope of the claims)

A cannabinoid film coating was prepared and applied to different types of tea leaves as follows. An emulsion was prepared by combining the ingredients listed in Table 11 to produce a mean ($d_{50}$) droplet size less than 5 $\mu$m.

Table 11

| Ingredients | % wt. | Mass |
|---|---|---|
| Modified food starch | 21.2% | 106.2 g |
| Coconut oil | 11.6% | 57.8 g |
| CBD isolate | 4.5% | 22.5 g |
| 85% THC distillate | 5.2% | 26.0 g |
| Rosemary extract | 0.017% | 0.09 g |
| Water | 57.5% | 287.5 g |
| Total | 100.0% | 500.0 g |

The emulsion was applied to a ~4.5 kg sample of each of three kinds of tea leaves: black, green, and mint. Tumble coating was used to apply the coating to each sample. In each case, the tea leaves were tumbled at 6-15 rpm while the liquid was applied intermittently. The film coatings provided a THC dose of about 1-2 mg per 1 gram of tea.

Example 10 (reference)

Water-soluble cannabinoid powders were prepared by spray drying. In each case, an emulsion was prepared from water soluble agents, carrier oil, CBD isolate, and water. The emulsion was then spray dried to produce a water soluble powder comprising about 2.5% by weight CBD (Compositions 10A and 10B) or about 25% by weight CBD (Compositions 10C and 10D). The chemical composition of each powder is listed in Table 12, wherein Compositions 10A and 10C were prepared with maltodextrin, and Compositions 10B and 10D were prepared with sorbitol. Additional compositions were prepared with THC distillate in place of the CBD isolate.

Table 12

| Ingredients | Compositions 10A and 10B | Compositions 10C and 10D |
|---|---|---|
| | % wt. | % wt. |
| Modified food starch | 6.2% | 32.0% |
| Maltodextrin or sorbitol | 86.6% | 15.2% |
| Coconut oil | 4.7% | 27.8% |
| CBD isolate | 2.5% | 25.0% |
| Total | 100.0% | 100.0% |

Example 11

Water soluble cannabinoid compositions (Compositions 11A, 11B, and 11C) in the form of agglomerated particles were prepared as follows. In each case, an emulsion was prepared by mixing modified food starch, sorbitol, MCT oil, purified cannabinoid mixture comprising THC distillate (about 87% by weight THC) and/or CBD isolate. The liquid emulsion was introduced in an agglomeration unit and atomized for contact with sorbitol particles. The chemical compositions of the agglomerated powders are listed in Table 13.

Table 13

| Ingredients | Composition 11A | | Composition 11B | | Composition 11C | |
|---|---|---|---|---|---|---|
| | % wt. | Mass (g) | % wt. | Mass (g) | % wt. | Mass (g) |
| Modified food starch | 6.3% | 524.0 g | 7.17% | 383.8 g | 5.8% | 671.2 g |
| MCT oil | 4.2% | 349.3 g | 4.8% | 255.9 g | 3.9% | 447.4 g |
| THC distillate | 1.7% | 145.5 g | 3.7% | 196.8 g | 0.2% | 19.2 g |
| CBD isolate | 1.5% | 123.2 g | -- | -- | 2.8% | 325.0 g |
| Sorbitol | 86.3% | 7216.3 g | 84.3% | 4,484.4 g | 87.3% | 10,037.2 g |
| Total | 100.0% | 8,358.3 g | 100.0% | 5320.9 g | 100.0% | 11,500 g |

Other aspects and embodiments of the present disclosure will be apparent to those skilled in the art from consideration of the specification and practice of the embodiments disclosed herein.

## Claims

**1.** A water soluble cannabinoid composition comprising:

a purified cannabinoid mixture;
at least one carrier oil; and
at least one water soluble agent chosen from a complex carbohydrate, a polyol, a polysaccharide, an oligosaccharide, or a combination thereof;
wherein the composition is soluble in water at a temperature less than or equal to about 20°C,
the composition being in the form of agglomerated particles.

2. The composition of claim 1, wherein the purified cannabinoid mixture is derived from cannabis plant matter and comprises less than 8.0%, less than 5.0%, or less than 1.0% by weight terpene compounds, such as from 0.1% to 5.0% by weight or from about 0.5% to about 3.0% by weight terpene compounds, optionally wherein the terpene compounds are chosen from β-myrcene, β-caryophyllene, limonene, linalool, α-bisabolol, α-pinene, β-pinene, caryophyllene oxide, terpinolene, phytol, or combinations thereof.

3. The composition of claim 1 or 2, wherein the purified cannabinoid mixture comprises at least 50% by weight, at least 70% by weight, or at least 85% by weight tetrahydrocannabinol (THC), cannabidiol (CBD), or a mixture thereof, and/or wherein the purified cannabinoid mixture comprises one or more of tetrahydrocannabivarin, cannabivarin, cannabigerol, cannabichromene, or cannabinol.

4. The composition of any of claims 1-3, wherein the composition comprises from 0.05% to 60% by weight of the purified cannabinoid mixture, such as from about 1.0% to about 50.0% by weight, from about 5.0% to about 40.0% by weight, from about 0.05% to about 35.0% by weight, or from about 10.0% to about 30.0% by weight of the purified cannabinoid mixture.

5. The composition of any of claims 1-4, wherein the at least one water soluble agent comprises a starch such as a modified food starch, gum arabic, quillaja extract, a cyclodextrin, a sugar alcohol such as sorbitol or maltitol, maltodextrin, or a combination thereof, and/or wherein the composition further comprises at least one antioxidant, flavoring agent, sweetener, coloring agent, food preservative, or combination thereof.

6. The composition of any of claims 1-5, wherein the composition is in the form of agglomerated particles having a bulk density ranging from 0.2 g/cm$^3$ to 0.7 g/cm$^3$ or from 0.4 g/cm$^3$ to 0.7 g/cm$^3$.

7. The composition of any of claims 1-6, wherein at least 40% by weight, such as at least 50% or at least 60% by weight, of the agglomerated particles have a particle size between about 150 μm and about 800 μm, and/or wherein at least 60% by weight of the agglomerated particles have a particle size between 150 μm and 600 μm.

8. The composition of any of claims 1-7, wherein the composition has a Hausner ratio less than or equal to 1.25 and/or a compressibility index less than or equal to 20.

9. The composition of any of claims 1-8, wherein 400 mg of the composition dissolves in water at a temperature less than or equal to 20°C within 30 seconds, within 20 seconds, or within 10 seconds, optionally wherein the composition is more soluble than sucrose.

10. The composition of any of claims 1-9, wherein the composition is sugar-free, nut-free, dairy-free, and/or gluten-free.

11. A food product or beverage product comprising the composition of any of claims 1-10.

12. A package comprising the composition of any of claims 1-10, or the food product or beverage product of claim 11, wherein the composition provides a cannabinoid dosage ranging from 0.5 mg/serving to 50.0 mg/serving, such as 1.0 mg/serving, 2.5 mg/serving, or 10 mg/serving, optionally wherein the package comprises a sachet, a packet, a canister, or a bottle.

13. The composition of any of claims 1-10, or the food product or beverage product of claim 11, for use in reducing pain, reducing nausea, reducing inflammation, reducing stress, promoting sleep, preparing for and/or recovering from exercise, or boosting energy.

## Patentansprüche

1. Eine wasserlösliche Cannabinoid-Zusammensetzung, die umfasst:

   eine gereinigte Cannabinoidmischung;
   mindestens ein Trägeröl; und
   mindestens ein wasserlösliches Mittel, ausgewählt aus einem komplexen Kohlenhydrat, einem Polyol, einem Polysaccharid, einem Oligosaccharid oder einer Kombination davon;
   wobei die Zusammensetzung in Wasser bei einer Temperatur von etwa 20°C oder weniger löslich ist,

wobei die Zusammensetzung in Form von agglomerierten Teilchen vorliegt.

2. Zusammensetzung nach Anspruch 1, wobei die gereinigte Cannabinoid-Mischung aus Cannabis-Pflanzenmaterial stammt und weniger als 8,0 Gew.-%, weniger als 5,0 Gew.-% oder weniger als 1,0 Gew.-% Terpenverbindungen, wie 0,1 bis 5,0 Gew.-% oder etwa 0,5 bis 3,0 Gew.-% Terpenverbindungen enthält, wobei die Terpenverbindungen gegebenenfalls aus β-Myrcen, β-Caryophyllen, Limonen, Linalool, α-Bisabolol, α-Pinen, β-Pinen, Caryophyllenoxid, Terpinolen, Phytol oder Kombinationen davon ausgewählt sind.

3. Zusammensetzung nach Anspruch 1 oder 2, wobei das gereinigte Cannabinoidgemisch mindestens 50 Gew.-%, mindestens 70 Gew.-% oder mindestens 85 Gew.-% Tetrahydrocannabinol (THC), Cannabidiol (CBD) oder ein Gemisch davon umfasst, und/oder wobei das gereinigte Cannabinoidgemisch eines oder mehrere von Tetrahydrocannabivarin, Cannabivarin, Cannabigerol, Cannabichromen oder Cannabinol umfasst.

4. Zusammensetzung nach einem der Ansprüche 1 bis 3, wobei die Zusammensetzung 0,05% bis 60 Gew.-% der gereinigten Cannabinoidmischung umfasst, wie etwa 1,0 bis etwa 50,0 Gew.-%, etwa 5,0 bis etwa 40,0 Gew.-%, 0,05 bis etwa 35,0 Gew.-% oder etwa 10,0 bis etwa 30,0 Gew.-% der gereinigten Cannabinoidmischung.

5. Zusammensetzung nach einem der Ansprüche 1 bis 4, wobei das mindestens eine wasserlösliche Mittel eine Stärke wie eine modifizierte Lebensmittelstärke, Gummiarabikum, Quillaja-Extrakt, ein Cyclodextrin, einen Zuckeralkohol wie Sorbit oder Maltit, Maltodextrin oder eine Kombination davon umfasst und/oder wobei die Zusammensetzung ferner mindestens ein Antioxidationsmittel, einen Geschmacksstoff, einen Süßstoff, ein Färbemittel, ein Lebensmittelkonservierungsmittel oder eine Kombination davon umfasst.

6. Zusammensetzung nach einem der Ansprüche 1 bis 5, wobei die Zusammensetzung in Form von agglomerierten Teilchen mit einer Schüttdichte im Bereich von 0,2 $g/cm^3$ bis 0,7 $g/cm^3$ oder von 0,4 $g/cm^3$ bis 0,7 $g/cm^3$ vorliegt.

7. Zusammensetzung nach einem der Ansprüche 1 bis 6, wobei mindestens 40 Gew.-%, wie mindestens 50 Gew.-% oder mindestens 60 Gew.-%, der agglomerierten Teilchen eine Teilchengröße zwischen etwa 150 μm und etwa 800 μm haben und/oder wobei mindestens 60 Gew.-% der agglomerierten Teilchen eine Teilchengröße zwischen 150 μm und 600 μm haben.

8. Zusammensetzung nach einem der Ansprüche 1 bis 7, wobei die Zusammensetzung ein Hausner-Verhältnis von weniger als oder gleich 1,25 und/oder einen Kompressibilitätsindex von weniger als oder gleich 20 aufweist.

9. Zusammensetzung nach einem der Ansprüche 1-8, wobei sich 400 mg der Zusammensetzung in Wasser bei einer Temperatur von 20°C oder weniger innerhalb von 30 Sekunden, innerhalb von 20 Sekunden oder innerhalb von 10 Sekunden auflösen, wobei die Zusammensetzung gegebenenfalls besser löslich ist als Saccharose.

10. Zusammensetzung nach einem der Ansprüche 1-9, wobei die Zusammensetzung zuckerfrei, nussfrei, milchfrei und/oder glutenfrei ist.

11. Lebensmittelprodukt oder Getränkeprodukt, das die Zusammensetzung nach einem der Ansprüche 1-10 enthält.

12. Verpackung, die die Zusammensetzung nach einem der Ansprüche 1-10 oder das Lebensmittelprodukt oder Getränkeprodukt nach Anspruch 11 enthält, wobei die Zusammensetzung eine Cannabinoid-Dosierung im Bereich von 0,5 mg/Portion bis 50,0 mg/Portion, wie 1,0 mg/Portion, 2,5 mg/Portion oder 10 mg/Portion, bereitstellt, wobei die Verpackung gegebenenfalls einen Beutel, ein Paket, einen Kanister oder eine Flasche umfasst.

13. Zusammensetzung nach einem der Ansprüche 1-10 oder Lebensmittel- oder Getränkeprodukt nach Anspruch 11 zur Verwendung bei der Verringerung von Schmerzen, der Verringerung von Übelkeit, der Verringerung von Entzündungen, der Verringerung von Stress, der Förderung des Schlafs, der Vorbereitung auf und/oder der Erholung von körperlicher Betätigung oder der Steigerung der Energie.

## Revendications

1. Composition de cannabinoïde hydrosoluble comprenant :

un mélange purifié de cannabinoïdes ;
au moins un véhicule huileux ; et
au moins un agent hydrosoluble choisi parmi un hydrocarbone complexe, un polyol, un polysaccharide, un oligosaccharide, ou une combinaison de ceux-ci ;
où la composition est soluble dans l'eau à une température inférieure ou égale à environ 20°C,
la composition étant sous la forme de particules agglomérées.

**2.** Composition selon la revendication 1, dans laquelle le mélange purifié de cannabinoïdes est dérivé d'une matière végétale de cannabis et comprend moins de 8,0%, moins de 5,0%, ou moins de 1,0% en masse de composés terpéniques, tel que de 0,1% à 5,0% en masse ou d'environ 0,5% à environ 3,0% en masse de composés terpéniques, éventuellement les composés terpéniques sont choisis parmi β-myrcène, β-caryophyllène, limonène, linalool, α-bisabolol, α-pinène, β-pinène, oxyde de caryophyllène, terpinolène, phytol, ou les combinaisons de ceux-ci.

**3.** Composition selon la revendication 1 ou 2, dans laquelle le mélange purifié de cannabinoïdes comprend au moins 50% en masse, au moins 70% en masse, ou au moins 85% en masse de tétrahydrocannabinol (THC), de cannabidiol (CBD), ou d'un mélange de ceux-ci, et/ou dans laquelle le mélange purifié de cannabinoïdes comprend un ou plusieurs de tétrahydrocannabivarine, cannabivarine, cannabigérol, cannabichromène, ou cannabinol.

**4.** Composition selon l'une des revendications 1 à 3, dans laquelle la composition comprend de 0,05% to 60% en masse de mélange purifié de cannabinoïdes, tel qu'environ 1,0% à environ 50,0% en masse, environ 5,0% à environ 40,0% en masse, environ 0,05% à environ 35,0% en masse, ou environ 10,0% à environ 30,0% en masse de mélange purifié de cannabinoïdes.

**5.** Composition selon l'une des revendications 1-4, dans laquelle le au moins un agent hydrosoluble comprend un amidon tel qu'un amidon alimentaire modifié, de la gomme arabique, un extrait de quillaja, une cyclodextrine, un polyol tel que le sorbitol ou le maltitol, de la maltodextrine, ou une combinaison de ceux-ci, et/ou dans laquelle la composition comprend en outre au moins un antioxydant, un agent aromatisant, un édulcorant, un colorant, un conservateur alimentaire, ou une combinaison de ceux-ci.

**6.** Composition selon l'une des revendications 1-5, dans laquelle la composition est sous la forme de particules agglomérées ayant une masse volumique apparente de 0,2 g/cm$^3$ à 0,7 g/cm$^3$ ou de 0,4 g/cm$^3$ à 0,7 g/cm$^3$.

**7.** Composition selon l'une des revendications 1-6, dans laquelle au moins 40% en masse, tel que au moins 50% ou au moins 60% en masse, des particules agglomérées ont une taille de particule entre environ 150 μm et environ 800 μm, et/ou dans laquelle au moins 60% en masse des particules agglomérées ont une taille de particule entre 150 μm et 600 μm.

**8.** Composition selon l'une des revendications 1-7, dans laquelle la composition a un indice de Hausner inférieur ou égal à 1,25 et/ou un indice de compressibilité inférieur ou égal à 20.

**9.** Composition selon l'une des revendications 1-8, dans laquelle 400 mg de la composition se dissolvent dans l'eau à une température inférieure ou égale à 20°C en 30 secondes, en 20 secondes, ou en 10 secondes, éventuellement dans laquelle la composition est plus soluble que le sucrose.

**10.** Composition de l'une des revendications 1-9, dans laquelle la composition est exempte de sucre, de noix, de produits laitiers et/ou de gluten.

**11.** Produit alimentaire ou boisson comprenant la composition de l'une des revendications 1-10.

**12.** Emballage comprenant la composition de l'une des revendications 1-10, ou le produit alimentaire ou la boisson de la revendication 11, dans lequel la composition fournit un dosage en cannabinoïdes de 0,5 mg/portion à 50,0 mg/portion, tel que 1,0 mg/portion, 2,5 mg/portion ou 10 mg/portion, l'emballage comprenant éventuellement un sachet, un paquet, une boîte ou une bouteille.

**13.** Composition de l'une des revendications 1-10, ou produit alimentaire ou boisson de la revendication 11, pour utilisation pour réduire la douleur, réduire la nausée, réduire l'inflammation, réduire le stress, favoriser le sommeil, pour se préparer à et/ou récupérer d'un exercice, ou pour stimuler l'énergie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017072762 A